# EUROPÄISCHE PATENTSCHRIFT

(11) **EP 3 086 825 B1**
(45) Veröffentlichungstag und Bekanntmachung des Hinweises auf die Patenterteilung: **02.09.2020**
(21) Anmeldenummer: 15723838.7
(22) Anmeldetag: 15.05.2015
(51) Int. Cl.: A61M 1/36, A61B 5/00, G01L 19/14

(54) **MEDIZINTECHNISCHES MESSSYSTEM UND VERFAHREN ZUR HERSTELLUNG DES MESSSYSTEMS**
MEDICAL MEASURING SYSTEM AND METHOD FOR THE PRODUCTION OF THE MEASUREMENT SYSTEM
SYSTÈME DE MESURE MÉDICAL ET PROCÉDÉ DE FABRICATION DU SYSTÈME DE MESURE

(30) Priorität: 15.05.2014 EP 14001734
(43) Veröffentlichungstag der Anmeldung: 02.11.2016
(73) Patentinhaber: novalung GmbH, 74076 Heilbronn (DE)
(72) Erfinder: MAURER, Andreas, 72072 Tübingen (DE); FILIPON, Sven, 74080 Heilbronn (DE); BEURER, Matthias, 70469 Stuttgart (DE)
(74) Vertreter: Graf von Stosch, Andreas
(86) Internationale Anmeldenummer: PCT/EP2015/001000
(87) Internationale Veröffentlichungsnummer: WO 2015/172890

(56) Entgegenhaltungen:
- EP-A1- 0 330 891
- WO-A1-97/15228
- WO-A1-2012/127422
- DE-U1- 29 810 331
- US-A- 5 581 038
- US-A1- 2005 284 815

## Beschreibung

Die vorliegende Erfindung betrifft ein medizintechnisches Messsystem mit einer Messvorrichtung zur Messung einer Eigenschaft eines Fluids, insbesondere zur Druckmessung, wobei die Messvorrichtung insbesondere umfasst: eine Leitung, die sich entlang einer Mittenlängsachse erstreckt und eingerichtet ist, ein Fluid, insbesondere Blut, innerhalb einer von einer Wandung umgrenzten Längskavität zu führen; und eine Sensoreinrichtung mit einem Sensor, die eingerichtet ist, eine Eigenschaft des in der Längskavität geführten Fluids zu messen. Ferner betrifft die vorliegende Erfindung ein Verfahren zur Herstellung eines solchen medizintechnischen Messsystems. Die vorliegende Erfindung betrifft insbesondere ein medizintechnisches Messsystem mit einzelnen Merkmalen des Anspruchs 1 sowie ein Verfahren mit einzelnen Merkmalen des unabhängigen Verfahrensanspruchs.

Es sind Messvorrichtungen oder Sensoren bekannt, welche an einem Konnektor, Adapter oder an einem Zwischenstück zwischen einzelnen Abschnitten einer Leitung angeordnet sind. Die Leitung führt ein Fluid, insbesondere eine isotonische Kochsalzlösung oder andere kristalloide Infusionslösungen oder Blut, dessen Eigenschaft, z.B. Druck erfasst werden soll. Bei solchen Messvorrichtungen besteht häufig das Risiko von Gerinnung (oder so genanntes Blut-Clotting) oder Hämolyse, insbesondere aufgrund irgendwelcher Kanten, Hinterschneidungen oder strömungstechnisch ungünstigen Übergängen. Die Verbindungselemente oder Konnektoren erhöhen auch das Risiko von Leckagen und müssen zusätzlich geprüft werden. Leckagen können dabei insbesondere an den Schnittstellen zwischen einem Konnektor und einem extrakorporalem Leitungs-/Schlauchset oder im Messsystem auftreten. Auch muss der Konnektor oder Adapter an der Leitung montiert werden, und es muss eine möglichst sterile oder sterilisierbare Abdichtung, Verklebung oder sonstige Schnittstelle sichergestellt werden können.

Alternativ zu solchen in die Leitung eingekuppelten oder eingebundenen Messvorrichtungen oder Sensoren kann eine Messung auch außerhalb der Leitung erfolgen. Der entsprechende Sensor kann einen Druck dann mittelbar erfassen, z.B. mittels einer Wassersäule. Bei dieser Art Messung ist jedoch ein so genannter Priming-Vorgang erforderlich, um die Messung starten zu können. Beim Priming wird das Leitungs-/Schlauchset mit einer kristalloiden Lösung wie z.B. isotoner Kochsalzlösung (NaCl) gefüllt, und daraufhin erfolgt eine vollständige Entlüftung eines Systems. Es besteht das Risiko, dass die Entlüftung des Leitungs-/Schlauchsystems fehlerhaft durchgeführt wird. Durch während der Messung in die kristalloide Lösung in zu einem Messsensor führende Leitungsabschnitte eindringendes Blut und dessen Gerinnung wird der Messvorgang beeinträchtigt oder unterbrochen, was lebensgefährlich sein kann.

Auch nachteilig bei dieser Art Messung sind Messfehler, die aufgrund von Gaseinschlüssen in der Wassersäule hervorgerufen werden. Auch kann die Messung selbst meist nur verzögert durchgeführt werden, da eine Druckwelle zunächst durch das Wasser übertragen werden muss. Die mittelbare Messung erschwert dadurch z.B. eine Synchronisation einer Pumpe mit der arteriellen Druckkurve des Patienten.

Abgesehen von der Frage, mittels welcher Vorrichtung der Druck gemessen werden soll, muss die Messvorrichtung technisch auch so ausgestaltet sein, dass die Messvorrichtungen auf robuste oder zuverlässige und fluiddichte Art an einer Leitung befestigt werden kann. Eine sichere Befestigung ist insbesondere dann erforderlich, wenn die Messvorrichtung nicht in Verbindung mit einem Konnektor, Adapter oder einem Zwischenstück der Leitung angeordnet werden kann oder soll.

Aus der DE 29810331 U1 ist ein Druckmesswandler für Blutleitungen in der Dialyse bekannt, der aus einem Basisteil und einem Deckelabschnitt besteht. Die beiden Abschnitte sind miteinander verbunden. Der zentrale Bereich des Basisteils ist vorzugsweise verformbar und die Basis und der Deckelabschnitt bestehen vorzugsweise aus PVC.

Aus der EP 0330891 A1 ist eine Anordnung zur Druckübertragung mit einem Außengehäuse bekannt, die mittels einer Membran in zwei separate Räume aufgeteilt ist. Die Anordnung weist eine Einlassöffnung für die Durchführung eines ersten Fluids auf, die zu einem der Räume führt.

Eine Verbindungsöffnung für ein zweites Fluid verbindet den anderen dieser Räume mit einer Druckerfassungsvorrichtung. Die Anordnung weist eine Membran auf, die fest an zumindest einem Teil des Umfangs der Einlassöffnung angeordnet ist, derart, dass die Einlassöffnung mit nur einem der Räume kommuniziert, während die Verbindungsöffnung nur mit dem anderen der beiden Räume kommuniziert. Die Anordnung ist dabei in erster Linie für die Drucküberwachung von Blut in Verbindung mit der Dialyse oder ähnlichen extrakorporalen Blutbehandlungen bestimmt.

Aus der WO 2012/127422 A1 ist eine Vorrichtung zur extrakorporalen Überwachung von physikalisch-chemischen Parametern von Blut bekannt. Die Vorrichtung umfasst einen Körper mit einem Einlass und einem Auslass für die organische Flüssigkeit, wobei mindestens eine innere Detektionskammer in dem Körper zwischen dem Einlass und dem Auslass ausgebildet ist. Der Körper umfasst dabei zumindest ein erstes Gehäuse für ein erstes Sensorelement zum Erfassen eines Druckparameters der Flüssigkeit und ferner zumindest ein zweites Gehäuse für ein zweites Sensorelement zum Erfassen eines Parameters, der nicht der Druckparameter ist.

Es ist eine Aufgabe der vorliegenden Erfindung, ein Messsystem bereitzustellen, bei welchem eine Messvorrichtung auf einfache und/oder sichere, robuste Weise an einer Leitung, insbesondere an einer flexiblen Leitung, flüssigkeitsdicht befestigt und positioniert werden kann. Dabei besteht die Aufgabe auch darin, ein Verfahren bereitzustellen, mittels welchem die Messvorrichtung auf einfache oder besonders zweckdienliche Weise an einer Leitung, z.B. an einem Schlauch eines extrakorporalen blutführenden Schlauchsystems, insbesondere an einer flexiblen Leitung, fixiert werden kann, um ein Messsystem zu bilden. Die Messvorrichtung kann dabei mehrere Komponenten einschließlich eines Sensors und eines oder mehrerer Kabel umfassen.

Die Erfindung geht aus von einem medizintechnischen Messsystem mit einer Messvorrichtung zur Messung einer Eigenschaft eines Fluids, insbesondere zur Druckmessung, wobei die Messvorrichtung umfasst:
- eine Leitung, insbesondere Schlauchleitung, die sich entlang einer Mittenlängsachse erstreckt und eingerichtet ist, ein Fluid, insbesondere Blut, innerhalb einer von einer Wandung umgrenzten insbesondere zylindrischen Längskavität zu führen;
- eine Sensoreinrichtung mit einem Sensor, die eingerichtet ist, eine Eigenschaft des in der Längskavität geführten Fluids zu messen.

Erfindungsgemäß ist vorgesehen, dass das Messsystem eine Umspritzung aufweist, welche die Messvorrichtung zumindest teilweise umgibt und wobei die Umspritzung sowohl zumindest die Sensoreinrichtung als auch einen Abschnitt der Leitung umgibt. Das erfindungsgemäße Messsystem weist ein mit der Sensoreinrichtung verbundenes Adapterkabel auf, welches in die Umspritzung eingebettet ist.

Durch diese erfindungsgemäße Maßnahme wird erreicht, dass die Sensoreinrichtung direkt an der Leitung fluiddicht befestigt und positioniert werden kann. Auf zusätzliche Komponenten wie z.B. einen Adapter, ein Zwischenstück oder einen Konnektor zum Anbringen der Sensoreinrichtung an der Leitung, kann dabei vorteilhafterweise verzichtet werden.

Die Umspritzung kann dabei ein Gehäuse für das Messsystem oder zumindest für einzelne Komponenten des Messsystems bilden. Bevorzugt weist die Umspritzung zumindest abschnittsweise eine umlaufend geschlossene Kontur auf, insbesondere eine umlaufende Mantelfläche, wodurch die Umspritzung auf stabile oder robuste Weise mit der Leitung verbunden werden kann. Gemäß einer Variante weist die Umspritzung konvex gewölbte Flächenabschnitte auf, insbesondere in mehreren Ebenen konvex gewölbte Flächenabschnitte, speziell in einer Ebene orthogonal zur Mittenlängsachse und auch in einer Ebene parallel zur Mittenlängsachse. Dies ermöglicht eine dreidimensionale Außenkontur, welche auf zweckdienliche Weise an eine Außenmantelfläche einer Leitung integriert werden kann. Die radialen Abmessungen können dabei im jeweiligen Abschnitt der Umspritzung minimiert werden, und die Umspritzung kann auch in ergonomischer Hinsicht optimiert werden. Dabei können alle Flächenabschnitte konvex (nach außen) gewölbt sein.

Die Umspritzung kann z.B. durch ein Klebeverfahren oder ein Umspritzverfahren oder ein Gießverfahren vorgesehen werden. Die Umspritzung kann dabei einen mechanischen Schutz der Sensoreinrichtung sicherstellen. Bevorzugt ist die Umspritzung blut- und/oder wasserabweisend ausgebildet.

Als Messvorrichtung ist dabei bevorzugt eine Vorrichtung zu verstehen, welche ein bestimmtes Fluid in einem bestimmten Zustand, insbesondere in einem Strömungszustand, führen bzw. zu- oder ableiten kann und eine Eigenschaft, z.B. den Druck, des Fluids erfassen und wahlweise auch zumindest teilweise auswerten kann. Eine solche Messvorrichtung kann z.B. für eine invasive Druckmessung verwendet werden, oder in Verbindung mit einem extrakorporalen Kreislauf, z.B. zum Nierenersatz, zur Herz-Lungen-Unterstützung oder Leberunterstützung, oder zur Messung des Infusionsdrucks oder Injektionsdrucks in fluidführenden Medizinprodukten. Speziell bei einer invasiven/implantierten Anordnung können sich dabei auch die Vorteile einer guten Dichtheit und/oder Sterilität ergeben. Neben einem Sensor kann die Messvorrichtung weitere Komponenten wie z.B. eine Schutzkappe, eine Steckerbuchse oder eine bevorzugt wasserabweisende Membran aufweisen, wobei die Membran einen Schutz des Sensors vor externen Einflüssen sicherstellen kann. Mittels der Messvorrichtung können z.B. Vitalfunktionen eines Patienten überwacht werden, z.B. Herzmuskelkontraktionen (Hämodynamik), oder es kann ein durch extrakorporale Zirkulation bedingter Druckverlust gemessen werden.

Als Fluid ist dabei bevorzugt eine Flüssigkeit zu verstehen, jedoch kann das Fluid auch ein Gas sein oder zumindest gasförmige Bestandteile aufweisen. Als eine Eigenschaft des Fluids kann dabei z.B. eine physikalische oder chemische Größe des Fluids oder eine den Zustand des Fluids beschreibende Größe verstanden werden. Eine Eigenschaft kann z.B. durch einen bestimmten Anteil einer gasförmigen Komponente beschrieben werden, z.B. einem Volumenanteil an CO2 oder O2.

Als Leitung ist dabei bevorzugt jede Art von Leitung zu verstehen, welche in Verbindung mit einer medizinischen Versorgung, Diagnose oder Therapie genutzt werden kann, z.B. auch in Verbindung mit irgendwelchen Kathetern. Die Leitung kann dabei auch Teil eines medizintechnischen Instruments oder Bestecks sein. Die insbesondere blutführende Leitung kann Teil eines so genannten Leitungs-/Schlauchsets sein oder dieses Leitungs-/Schlauchset bilden. Die Leitung kann dabei auch eine den Zugang zum Körper sicherstellende Kanüle umfassen oder abschnittsweise als Kanüle ausgebildet sein. Die Leitung kann auch durch einen anderen fluidführenden Hohlkörper gebildet sein. Bevorzugt ist die Leitung zumindest abschnittsweise flexibel, also elastisch formbar. Insbesondere kann die Leitung gekrümmt oder gebogen werden. Die Elastizität der Leitung wird dabei durch die Sensoreinrichtung nicht oder nicht spürbar beeinflusst. Der Durchmesser der Leitung kann weitgehend frei gewählt werden. Zweckdienlich sind insbesondere Innendurchmesser von z.B. 3/8" oder 1/4".

Die Wandung oder auch die gesamte Leitung kann aus einem flexiblen Kunststoffmaterial, insbesondere aus Polyvinylchlorid (PVC)-Material ausgeführt sein. Bevorzugt ist das Kunststoffmaterial ein hochreines, phthalatfreies Weich-PVC. Im einfachsten Fall handelt es sich bei der Leitung z.B. um einen in der Medizintechnik häufig eingesetzten PVC-Schlauch. Eine Wandstärke der Wandung und/oder Leitung liegt dabei z.B. im Bereich von 1mm bis 5mm, bevorzugt im Bereich von 1.2mm bis 3.5mm, weiter bevorzugt im Bereich von 1.5mm bis 3mm, insbesondere im Bereich von 1.6mm bis 2.4mm.

Als Sensoreinrichtung ist dabei bevorzugt eine Komponente der Messvorrichtung zu verstehen, mittels welcher ein Messsignal, z.B. ein Drucksignal, erfasst und entweder weiterverarbeitet oder zumindest weitergeleitet werden kann.

Als Sensor ist dabei bevorzugt eine Komponente der Messvorrichtung zu verstehen, mittels welcher ein Messignal, z.B. ein Drucksignal zumindest erfasst werden kann. Beispielsweise kann ein piezoresistiver Sensor verwendet werden. Wahlweise können auch Sensoren eingesetzt werden, welche auf einem oder mehreren der folgenden physikalischen Prinzipien oder Funktionsweisen basieren: z.B. piezoelektrisch, kapazitiv, induktiv, frequenzanalog, oder Sensor mit Hallelement, faseroptischer Sensor. Der Sensor kann dabei an seiner dem Fluid/Blut zugewandten Fläche in die Kontur des Innenlumens der Leitung so eingepasst sein, dass ein stufenfreier und lückenloser Übergang zwischen dem Sensor und dem Innenlumen gewährleistet ist, insbesondere um Gerinnung und Hämolyse im Bereich des Sensors zu vermeiden.

Als Umspritzung ist dabei bevorzugt eine aus Füllmaterial gebildete Umhüllung oder ein Gehäuse zu verstehen, welches zumindest teilweise eine Außenoberfläche des Messsystems bereitstellt. Das Füllmaterial ist bevorzugt ein Material, welches mittels Spritzgussverfahren formbar ist, insbesondere mittels so genanntem "Hotmelt Moulding" oder "Macromelt Moulding" oder Niederdruck-Vergusstechnik.

Gemäß einem Ausführungsbeispiel ist die Umspritzung derart an der Leitung vorgesehen, dass die Umspritzung zumindest die Sensoreinrichtung oder auch zusätzliche Komponenten der Messvorrichtung umgibt, wobei zumindest die Sensoreinrichtung in einer vordefinierten Position relativ zur Leitung in der Umspritzung positioniert ist, insbesondere eingebettet ist. Hierdurch kann die Sensoreinrichtung zumindest teilweise auch durch die Umspritzung abgestützt werden, insbesondere nach radial außen, und die Schnittstelle zwischen Wandung und Sensoreinrichtung kann vergleichsweise robust ausgestaltet werden. Zwar kann die Sensoreinrichtung auch direkt an der Wandung fixiert werden. Die Umspritzung kann jedoch verhindern, dass externe Kräfte auf die Sensoreinrichtung einwirken, und dass eine vorbestimmte Soll-Position der Sensoreinrichtung wahlweise auch ohne irgendeine bestimmte Art der Verbindung, insbesondere ohne das Erfordernis einer stoffschlüssigen Verbindung, sichergestellt werden kann.

Gemäß einem Ausführungsbeispiel ist die Umspritzung aus einem Schmelzklebstoff gebildet. Die Verwendung eines Schmelzklebstoff liefert die im Folgenden näher beschriebenen Vorteile, sei es bei der Handhabung oder bei der Herstellung des Messsystems.

Gemäß einem Ausführungsbeispiel ist die Umspritzung aus einer Mischung aus Polyester und Kohlenwasserstoffharz ausgebildet. Hierdurch kann eine gute Anhaftung auf der Leitung sichergestellt werden, ferner eine gute Beständigkeit, und auch eine gute Haptik. Bevorzugt weist die Umspritzung einen größeren Anteil Polyester als Kohlenwasserstoffharz auf, insbesondere mindestens 60 von 100 Anteilen. Weiter bevorzugt ist die Umspritzung durch eine Mischung von mindestens 70 Anteilen Polyester gebildet. Gemäß einer bevorzugten Variante ist die Umspritzung durch eine Mischung von 75 bis 85 Anteilen Polyester und 15 bis 25 Anteilen Kohlenwasserstoffharz gebildet. Diese Mischung liefert insbesondere auch einen guten Kompromiss aus Fließverhalten und Trocknungsverhalten. Es hat sich gezeigt, dass ein Mischungsverhältnis von zumindest annähernd 80 Anteilen Polyester und zumindest annähernd 20 Anteilen Kohlenwasserstoffharz besonders vorteilhafte Materialeigenschaften sicherstellen kann. Ein Verhältnis von 80 zu 20 kann dabei für viele Anwendungen bevorzugt sein. Gemäß einer Variante kann die Mischung auch Farbpigmente aufweisen, insbesondere im Bereich von 1 bis 3 Anteilen, bevorzugt 1.5 Anteilen. Sind Farbpigmente vorgesehen, so kann der Anteil von Polyester und/oder Kohlenwasserstoffharz entsprechend reduziert sein. Dabei handelt es sich bevorzugt um Gewichtsanteile.

Es hat sich gezeigt, dass bei einer Mischung aus Polyester und Kohlenwasserstoffharz mittels des Polyesters eine gute Isopropanolbeständigkeit sichergestellt werden kann. Ferner kann Polyester eine gute Elastizität der Umspritzung im Endprodukt sicherstellen. Polyester weist im Verarbeitungs-Prozess eine vergleichsweise hohe Viskosität auf.

Es hat sich ferner gezeigt, dass bei einer Mischung aus Polyester und Kohlenwasserstoffharz mittels des Kohlenwasserstoffharzes eine gute Oberflächen-Haftung sichergestellt werden kann, insbesondere auf PVC-Schläuchen. Ferner kann Kohlenwasserstoffharz die Steifigkeit oder Formbeständigkeit der Umspritzung sicherstellen. Kohlenwasserstoffharz weist im Verarbeitungs-Prozess eine vergleichsweise hohe Viskosität auf.

Die Dichte der Umspritzung beträgt bevorzugt 0.9 bis 0.95 kg/m³. Die Oberfläche der Umspritzung ist bevorzugt glatt und weist keine Poren auf. Die Oberfläche ist bevorzugt geschlossen-porig.

Mit einem Schmelzklebstoff-Material kann insbesondere auch eine gute Haftung an PVC-Schläuchen sichergestellt werden. Es hat sich gezeigt, dass dieses Umspritzmaterial im Vergleich zu Polyamid-basierten Hotmelts eine besonders gute Haftung an PVC-Schläuchen und auch eine besonders gute Isopropanolbeständigkeit aufweist. Die Isopropanolbeständigkeit ist insofern vorteilhaft, als die Umspritzung mit herkömmlichen Desinfektionsmitteln desinfiziert werden kann, ohne dass die Oberfläche sich verändert. Die Oberfläche der Umspritzung bleibt bei Kontakt mit Desinfektionsmitteln beständig. Sie wird nicht kleberig und zersetzt sich auch nicht. Auch gegenüber Materialien auf Basis von Synthesekautschuk oder synthetischen Polymeren konnten diese Vorteile festgestellt werden. Es hat sich zudem gezeigt, dass dieses Umspritzmaterial eine gute Elastizität aufweist, insbesondere bei Wandstärken bis etwa 1.4mm.

Das Umspritzmaterial kann z.B. in Granulat-Form bereitgestellt werden. Das Umspritzmaterial kann z.B. in schwarzer Färbung oder Farbe vorliegen. Die Viskosität des Umspritzmaterials kann z.B. im Bereich von 20.000 ± 5.000 mPa●s liegen, gemessen nach DIN 53019 (bei 190°C). Der Schmelzpunkt des Umspritzmaterials kann z.B. bei 165°C +/- 3° liegen, gemessen in einer Kofler-Bank, also in einer Heiztischapparatur nach Kofler. Die offene Zeit kann bei ca. 40sec liegen. Das Umspritzmaterial kann ein Schrumpfverhalten von z.B. ca. -2% aufweisen.

Die Umspritzung kann dabei auch eine Dämpfungsfunktion erfüllen. Mit anderen Worten kann das Messsystem dank der Umspritzung auch besonders robust ausgeführt werden. Dabei kann die Umspritzung auch aus einem Material mit einer hohen Elastizität ausgebildet sein, insbesondere mit einem Anteil von mindestens 70 von 100 von Polyester, bevorzugt einem Anteil von 80 von 100. Die Elastizität der Umspritzung liefert auch den Vorteil, dass die Leitung flexibel bleibt, und dass sich die Umspritzung nicht ohne weiteres von der Leitung loslöst, selbst bei stärkeren Relativbewegungen oder längerer Einsatzdauer des Messsystems.

Gemäß einem Ausführungsbeispiel ist die Umspritzung eingerichtet, an der Außenmantelfläche der Wandung anzuhaften, insbesondere durch physikalische Bindung an die Wandung. Hierdurch kann die Umspritzung in einer eindeutigen Position relativ zur Leitung positioniert werden und dadurch auch andere Komponenten der Messvorrichtung positionieren. Eine chemische Verbindung des Umspritzmaterials mit der Wandung ist dabei nicht erforderlich. Vielmehr kann die Anhaftung bereits allein durch physikalische Verbindung sichergestellt werden, insbesondere mechanische Adhäsion. Es hat sich gezeigt, dass das physikalische Anhaften gut mit einem Umspritzmaterial aus Polyester und Kohlenwasserstoffharz sichergestellt werden kann, insbesondere auf PVC-Schläuchen.

Gemäß einem Ausführungsbeispiel umgibt die Umspritzung zumindest die Leitung zumindest abschnittsweise vollumfänglich, insbesondere an mindestens zwei voneinander beabstandeten Abschnitten der Umspritzung, bevorzugt an zwei in maximalem Abstand zueinander angeordneten freien Enden der Umspritzung. Hierdurch kann die Umspritzung auf sichere und robuste Weise mit der Leitung verbunden werden, insbesondere im Bereich eines proximalen und/oder distalen Endes oder Abschnitts der Umspritzung. Dabei kann die Umspritzung an einer Unterseite des Messsystems eine oder mehrere Aussparungen aufweisen. Eine Aussparung kann dabei zum einen eine verbesserte Flexibilität des Systems ermöglichen, selbst bei einem Umspritzmaterial mit einer vergleichsweise niedrigen Elastizität. Zum anderen kann eine Aussparung auch sicherstellen, dass der Sensor von der anderen Seite der Leitung (d.h., von der Unterseite der Umspritzung) zu sehen ist. Die Aussparung kann daher auch die Funktion eines Fensters erfüllen.

Gemäß einem Ausführungsbeispiel sind weitere Komponenten des Messsystems, insbesondere eine Steckerbuchse oder wahlweise auch ein Adapterkabel, relativ zur Leitung mittels der Umspritzung positioniert und in einer vorbestimmten Position relativ zueinander und/oder relativ zur Leitung in der Umspritzung gelagert, wobei die Umspritzung diese Komponenten zumindest teilweise umgibt (insbesondere radial außen) oder einbettet. Hierdurch ist es nicht erforderlich, die Steckerbuchse irgendwie an der Leitung zu fixieren. Die Steckerbuchse kann mittels der Umspritzung in Position gehalten werden. Das Adapterkabel und/oder die Steckerbuchse können zumindest teilweise von der Umspritzung umgeben sein.

Die Umspritzung kann dabei sowohl die Leitung als auch weitere Komponenten des Messsystems, abgesehen von einem optional vorgesehenen Taster zum Betätigen der Sensoreinrichtung, umgeben und ein integrales Gehäuse bilden, in welches die weiteren Komponenten integriert sind.

Gemäß einem Ausführungsbeispiel ist die Leitung unmittelbar direkt, insbesondere stoffschlüssig mit der Sensoreinrichtung verbunden, insbesondere mittels eines Haftmittels. Hierdurch kann eine eindeutige Position des Sensors über die Position der Sensoreinrichtung definiert werden, unabhängig von irgendwelchen elastischen Eigenschaften der Umspritzung. Dabei kann mindestens eine weitere Komponente des Messsystems, bei welchen eine exakte Positionierung nicht erforderlich oder gewünscht ist, mittelbar durch die Umspritzung mit der Leitung verbunden sein oder mittels der Umspritzung relativ zur Leitung positioniert sein.

Gemäß einem Ausführungsbeispiel weist die Umspritzung an einem proximalen und/oder distalen Ende oder Abschnitt der Umspritzung einen Fortsatz oder Rand auf, welcher an der Außenmantelfläche der Wandung zur Anlage kommt und eingerichtet, die Leitung auszurichten und innerhalb der Umspritzung zu stabilisieren. Hierdurch kann ein Knickschutz bereitgestellt werden. Der Fortsatz kann vermeiden, dass die Leitung dort, wo wie Umspritzung beginnt, besonders stark beansprucht wird oder sogar abknickt. Bevorzugt ist der Fortsatz oder Rand vollständig umlaufend ausgebildet. Weiter bevorzugt ist der Fortsatz oder Rand rohrförmig ausgebildet. Der Fortsatz weist bevorzugt eine Wandstärke bis max. 1.4mm auf, was eine gute Elastizität sicherstellen kann. Der Fortsatz weist bevorzugt eine Länge im Bereich von 5mm bis 20mm, weiter bevorzugt 7mm bis 15mm, besonders bevorzugt 8mm bis 10mm auf. Eine solche Länge kann sicherstellen, dass eine Biegung der Leitung derart in die Umspritzung weitergeleitet wird, dass die Umspritzung gut an der Leitung anhaftet, auch bei häufigen oder starken Relativbewegungen.

Gemäß einem Ausführungsbeispiel weist die Umspritzung einen sich bevorzugt in radialer Richtung, insbesondere zumindest annähernd senkrecht/orthogonal zur Mittenlängsachse erstreckenden Durchlass auf, wobei der Durchlass eine Verbindung (einen Kommunikationspfad) zwischen dem Sensor und der Umgebung bereitstellt. Der Sensor steht durch den Durchlass hindurch in Verbindung mit der Umgebung. Hierdurch kann die Umspritzung die Messvorrichtung im Wesentlichen vollständig einbetten und in mehreren Richtungen und Ebenen lagern und positionieren, ohne dass eine Verbindung des Sensors der Sensoreinrichtung zur Umgebung unterbrochen ist oder über eine zusätzliche Leitung oder Durchführung hergestellt werden muss. Bevorzugt erstreckt sich der Durchlass in radialer Richtung, insbesondere zumindest annähernd senkrecht/orthogonal zur Mittenlängsachse.

Gemäß einem Ausführungsbeispiel weist das Messsystem eine Steckerbuchse auf, wobei die Umspritzung die Steckerbuchse zumindest teilweise umgibt und bevorzugt auch zwischen der Leitung und der Steckerbuchse vorgesehen ist. Mit anderen Worten: Die Steckerbuchse ist auch an einer Unterseite in die Umspritzung eingebettet und allein mittels der Umspritzung relativ zur Leitung positioniert. Die Steckerbuchse kontaktiert die Leitung nicht direkt, sondern ist nur mittelbar über die Umspritzung mit der Leitung verbunden. Hierdurch kann vermieden werden, dass die Steckerbuchse bei einer Handhabung, insbesondere Biegung der Leitung an der Außenmantelfläche der Leitung reibt. Eine Relativbewegung zwischen einem vergleichsweise harten Material der Steckerbuchse und der Leitung kann weitgehend vermieden werden. Die Umspritzung kann als Dämpfungselement zwischen der Steckerbuchse und der Leitung wirken, was insbesondere bei Steckerbuchsen mit vergleichsweise großer Erstreckung zweckdienlich ist.

Gemäß einem Ausführungsbeispiel überlappt die Umspritzung die Sensoreinrichtung in Längsrichtung entlang der Mittenlängsachse in beiden Richtungen, wobei die Umspritzung bevorzugt eine Stirnseite aufweist, die in Längsrichtung zumindest annähernd bündig in einer Ebene mit einem stirnseitigen Ende einer Komponente des Messsystems angeordnet ist, insbesondere bündig mit der Sensoreinrichtung und/oder einer/der Steckerbuchse der Messvorrichtung. Hierdurch kann eine Anordnung bereitgestellt werden, bei welcher auch weitere Komponenten von der Umspritzung geschützt werden und derart in die Umspritzung eingebettet sind, dass die weiteren Komponenten nicht mit anderen Gegenständen verkanten oder an irgendwelchen aus der Umspritzung hervorstehenden Teilen beschädigt werden.

Das Messsystem weist ein mit der Sensoreinrichtung verbundenes Adapterkabel auf, welches in die Umspritzung eingebettet ist, wobei das Adapterkabel bevorzugt mit Überlänge, insbesondere in einer Schlangenlinie, in der Umspritzung angeordnet ist. Hierdurch kann die Sensoreinrichtung mit einer Steckerbuchse verbunden werden, ohne dass eine Relativbewegung zwischen der Steckerbuchse und der Sensoreinrichtung Zugspannungen auf die Sensoreinrichtung hervorruft. Bei einer solchen Anordnung kann die Leitung nach wie vor eine hohe Flexibilität aufweisen und wie üblich gehandhabt werden. Die Umspritzung kann aus einem vergleichsweise weichen Material ausgebildet sein, und Relativbewegungen zwischen den Komponenten des Messsystems können dank der Überlänge kompensiert werden. Zusätzliche Halterungen für das Kabel sind dabei entbehrlich. Eine Biegung der Leitung führt nicht zu Spannungen innerhalb des Messsystems. Hierdurch kann sichergestellt werden, dass die Sensoreinrichtung auch bei unachtsamer Handhabung der Leitung mit hohem Sicherheitsfaktor fluiddicht an der Leitung positioniert bleibt. Das Adapterkabel ermöglicht auch eine Einbettung der Steckerbuchse in die Umspritzung, ohne die Steckerbuchse zusätzlich auf der Leitung fixieren zu müssen.

Gemäß einem Ausführungsbeispiel ist die Umspritzung ergonomisch für die Innenfläche einer menschlichen Hand geformt, insbesondere mit mindestens einem konvexen Außenflächenabschnitt, der in Umfangsrichtung und in Bezug auf die Mittenlängsachse konvex gewölbt ist. Hierdurch kann die Leitung im Bereich der Umspritzung ergriffen und gehalten und gehandhabt werden. Selbst dünne Leitungen mit einem Durchmesser von z.B. 1/8Zoll können hierdurch auf ergonomische Weise gehandhabt werden. Dabei kann die Umspritzung auch eine in Bezug auf die Handhabung des Systems angepasste Geometrie aufweisen, oder eine Geometrie, welche auf die Fluss-Richtung des Bluts oder auf die Position der Sensoröffnung und des Steckverbinders hindeutet.

Gemäß einem Ausführungsbeispiel verjüngt sich die Umspritzung in mindestens einer Richtung entlang der Mittenlängsachse, insbesondere von einer steckerseitigen Rückseite hin zu einem vorderen Abschnitt, an welchem die Sensoreinrichtung angeordnet ist. Hierdurch kann die Menge erforderlichen Materials minimiert werden, insbesondere in Verbindung mit einem Spritzgußverfahren. Hierdurch kann einem Benutzer auch auf einen Blick angezeigt werden, in welcher Richtung die von der Umspritzung umhüllte Leitung das Fluid führt, was die Praktikabilität des Messsystems weiter erhöht.

Gemäß einem Ausführungsbeispiel weist die Leitung eine in radialer Richtung in die Wandung eingebrachte Radialkavität auf, in welcher zumindest der Sensor der Sensoreinrichtung angeordnet ist und derart in die Wandung integriert ist, dass der Sensor in Kommunikation mit dem in der Längskavität geführten Fluid ist. Hierdurch kann sichergestellt werden, dass die Sensoreinrichtung in Verbindung mit der Umspritzung zu einer Messeinheit verbunden werden kann. Die direkte Anordnung in der Wandung, d.h. die Integration in die Wandung, hat auch den Vorteil, dass die Flexibilität der Leitung nicht vermindert wird, und dass auf einfache Weise eine spezifische Position für die Messeinheit gewählt werden kann.

Ferner kann durch die Anordnung des Sensors der Sensoreinrichtung in der Radialkavität der Wandung auch eine mechanische Sicherung der Sensoreinrichtung und damit des Messsystems an der Leitung erreicht werden, die die stoffschlüssige Anbindung des Messsystems an der Leitung durch die Umspritzung in vorteilhafter Weise ergänzt.

Sensoren sind seit den letzten Jahren mit immer kleineren Abmessungen erhältlich. Dies ermöglicht eine vorteilhafte Anordnung des Sensors in unmittelbarer Nähe des zu messenden Fluids. Der integrierte Sensor kann direkt am Fluidstrom angeordnet werden, ohne den Fluidstrom zu beeinflussen. Dies ermöglicht auch eine besonders exakte Messung. Hierbei kann z.B. auch eine Integration in eine Wandung erfolgen, welche nur eine Wandstärke im Bereich von 1mm bis 3mm aufweist.

Eine solche Anordnung des Sensors ermöglicht auch eine weitgehend beliebige Auswahl der Position des Sensors an irgendeinem Abschnitt oder irgendeiner Umfangsposition entlang der Leitung.

Die in die Wandung integrierte Anordnung des Sensors macht Zwischenstücke, Kupplungen oder sonstige Anschlüsse entbehrlich. Es sind z.B. keine Luer-Anschlüsse oder andere Zugänge zur Leitung erforderlich. Hierdurch kann das Risiko von Leckstellen oder von irgendwelchen unsterilen Schnittstellen deutlich vermindert werden. Zudem kann eine Fehlbedienung irgendwelcher Anschlüsse vermieden werden. Es kann z.B. vermieden werden, dass z.B. in einem extrakorporalen Leitungs-/Schlauchsystem (wie z.B. bei der Dialyse, bei der Herz- oder Lungenunterstützung oder bei kardiopulmonalem Bypass in der Herzchirurgie, oder in einem Katheter bzw. einer Schleuse, z.B. in der Kardiologie) in einem Abschnitt mit negativem Druck (insbesondere kleinerer Druck als Atmosphärendruck) Luft im Bereich der Mess-Anordnung in das Leitungs-/Schlauchsystem bzw. in den Katheter eingezogen wird, oder dass in einem Abschnitt mit positivem Druck (insbesondere dort wo Blut in den Körper zurückgeführt wird) Fluid/Blut aus dem Kreislauf austritt.

Ein Priming-Vorgang speziell in Bezug auf den Sensor ist dabei nicht mehr erforderlich, was Zeit einsparen kann und die Messung in bestimmten (z.B. lebensbedrohlichen) Situationen besonders nützlich machen kann. Zusätzliche zu entlüftende Zuleitungen oder Schläuche liegen nicht mehr vor. Mit anderen Worten: Die integrierte Anordnung des Sensors ermöglicht eine "inline"-Messung ohne zeitliche Verzögerung. Das blutführende Leitungs-/(Schlauchsystem (einschließlich einem eventuell vorhandenen Katheter bzw. einer Kanüle) kann dabei unabhängig von einer Messung gefüllt und entlüftet werden.

Eine Messung kann dabei an unterschiedlichen Positionen ausgeführt werden. Insbesondere kann ein Druck dabei an mehreren Messpositionen gemessen werden, insbesondere in Abhängigkeit einer jeweiligen medizinischen Anwendung, z.B. ein Saugdruck an einer ersten Position vor einer Pumpe, ein Pumpendruck an einer zweiten Position nach der Pumpe, und ein weiterer an einer dritten Position, insbesondere ein Reperfusionsdruck nach einem Membranventilator. Mit anderen Worten: Die Messvorrichtung kann wahlweise auch eine Vielzahl von Sensoreinrichtungen oder zumindest eine Vielzahl von Sensoren aufweisen. Dementsprechend kann die Leitung oder der Katheter bzw. die Kanüle auch eine Vielzahl von radialen Radialkavitäten aufweisen.

Als Radialkavität ist dabei bevorzugt eine in radialer Richtung eingebrachte oder vorgesehene Ausnehmung, eine Bohrung, eine Aussparung oder auch ein ausgehöhlter Bereich oder Abschnitt oder Aufnahmevolumen zu verstehen. Die Radialkavität kann auch durch eine sich in radialer Richtung erstreckende Längskavität gebildet sein, welche nur von einer Seite der Wandung zugänglich ist. Die Radialkavität muss nicht notwendigerweise eine Durchführung oder ein Loch in der Wandung sein.

Als Öffnung ist dabei bevorzugt ein Durchlass oder eine Radialkavität zu verstehen, welche die Wandung vollständig durchbricht, also durchgehend durch die Wandung vorgesehen ist. Als Ausnehmung ist dabei bevorzugt eine sich in radialer Richtung von einer Innenseite der Wandung, also einer Innenmantelfläche, erstreckende Kavität zu verstehen, die nicht notwendigerweise bis zu einer Außenmantelfläche der Wandung verlaufen muss. Mit anderen Worten: Die Ausnehmung ist nicht notwendigerweise ein Loch in der Wandung, sondern kann die Wandung auch nur abschnittsweise in radialer Richtung aushöhlen.

Als eine Anordnung "in Kommunikation mit" ist dabei bevorzugt eine Anordnung zu verstehen, bei welcher der Sensor in direktem Kontakt mit dem Fluid ist. Der Sensor kann im Fluidstrom oder an der Seite des Fluidstroms oder an der Seite eines Strömungspfades des Fluidstroms angeordnet werden.

Die Sensoreinrichtung oder der Sensor können dabei einen Teil der Wandung bilden. Der Sensor kann an einem Außenrand der Längskavität angeordnet sein und die Längskavität in radialer Richtung begrenzen. Der Sensor kann sich zumindest annähernd über das gesamte Querschnittsprofil der Radialkavität erstrecken und zumindest annähernd dieselbe Ausdehnung aufweisen wie ein Durchmesser oder eine Ausdehnung der Radialkavität. Die Sensoreinrichtung kann eingerichtet sein, den Sensor in einer mittels der Radialkavität vordefinierten Radialposition zu positionieren.

Gemäß einem Ausführungsbeispiel bildet die Radialkavität zusammen mit der Sensoreinrichtung und/oder dem Sensor eine Übermaßpassung, an welcher die Wandung fluiddicht abdichtbar ist. Hierdurch kann diese Schnittstelle weitgehend unabhängig von irgendeiner stoffschlüssigen Verbindung abgedichtet werden. Als Übermaßpassung ist dabei bevorzugt eine Schnittstelle zu verstehen, bei welcher eine der zu kuppelnden Komponenten ein bestimmtes Aufmaß aufweist, durch welches sichergestellt werden kann, dass die beiden zu kuppelnden Komponenten nur bei einer geometrischen Anpassung aufeinander miteinander verbunden werden können, und dass eine jedenfalls spielfreie und spaltfreie Verbindung sichergestellt ist. Die Radialkavität kann z.B. eine Bohrung sein, die bevorzugt durchgehend durch die Wandung vorgesehen ist, insbesondere eine zylindrische Bohrung mit einheitlichem Durchmesser. Der Sensor oder ein radial nach innen vorstehendes freies Ende der Sensoreinrichtung kann bündig zu einer Innenmantelfläche der Wandung angeordnet sein. Hierdurch können günstige Strömungsverhältnisse sichergestellt werden. Durch Hinterschneidungen oder Kanten verursachte Verwirbelungen können weitgehend vermieden werden. Hierdurch kann auch ein Risiko von Blut-Gerinnseln oder Hämolyse verringert werden. Der Sensor kann dabei in einer Radialposition angeordnet sein, welche zumindest annähernd einem Radialabstand einer Innenmantelfläche der Leitung im Bereich der Radialkavität entspricht. Die Sensoreinrichtung kann einen Anlageabschnitt aufweisen, welcher zumindest im Bereich der Radialkavität, insbesondere umlaufend um die Radialkavität, geometrisch korrespondierend zu einer Außenmantelfläche der Leitung oder Wandung ausgebildet ist. Hierdurch kann die Sensoreinrichtung auf einfache und robuste Weise mit der Leitung verbunden werden, insbesondere umlaufend um den Bereich der Radialkavität an die Leitung angeklebt werden.

Als Anlageabschnitt ist dabei bevorzugt ein flächiger Abschnitt zu verstehen, an welchem die Sensoreinrichtung an der Leitung zur Anlage gebracht werden kann. Bevorzugt ist der Anlageabschnitt auch formstabil, also elastisch oder plastisch nicht verformbar, so dass eine Relativbewegung zwischen der Leitung und dem Anlageabschnitt im Bereich der Radialkavität vermieden werden kann. Dies kann eine dauerhaft, sichere Verbindung zwischen diesen beiden Komponenten sicherstellen. Der Anlageabschnitt kann z.B. eine konkave Kontur aufweisen, welche geometrisch korrespondierend zu einer konvexen Kontur der Leitung oder Wandung ausgebildet ist. Hierdurch kann zum einen eine verhältnismäßig robuste, belastbare flächige Verbindung zwischen der Leitung und dem Anlageabschnitt sichergestellt werden. Zum anderen kann effektiv vermieden werden, dass die Leitung relativ zur Sensoreinrichtung verdreht wird. Mit anderen Worten: diese korrespondierende Geometrie kann die Fluiddichtheit an der Schnittstelle zwischen dem Sensor und der Leitung sicherstellen, selbst wenn an dieser Schnittstelle kein Haftmittel sondern lediglich z.B. eine Übermaßpassung vorgesehen ist.

Der Anlageabschnitt kann zumindest im Bereich der Radialkavität, insbesondere umlaufend um die Radialkavität, stoffschlüssig mit einer/der Außenmantelfläche der Leitung oder Wandung verbunden sein, insbesondere mittels eines Haftmittels. Hierdurch kann die Sensoreinrichtung auf robuste Weise an der Leitung fixiert werden. Die stoffschlüssige Verbindung im Bereich der Außenmantelfläche hat auch den Vorteil, dass irgendein Haftmittel, insbesondere Kleber, nicht notwendigerweise an einer Innenfläche der Radialkavität vorgesehen sein muss. Hierdurch kann vermieden werden, dass das Fluid, insbesondere Blut, in Kontakt mit dem Haftmittel gelangt. Gemäß einer Variante kann die Sensoreinrichtung allein mittels des Umspritzmaterials mit der Wandung verbunden werden, insbesondere bei einem speziell dafür eingerichteten und geometrisch ausgebildeten Anlageabschnitt.

Die Sensoreinrichtung kann an einer Innenfläche der Radialkavität direkt ohne Haftmittel passgenau und fluiddicht zur Anlage gebracht sein. Die Innenfläche der Radialkavität kann z.B. zylindrisch sein oder einen polygonen Querschnitt aufweisen. Die Radialkavität ist nicht notwendigerweise rund oder kreisrund, sondern kann einen beliebigen Querschnitt aufweisen. Bevorzugt ist die Radialkavität kreisrund. Dies erleichtert eine passgenaue Anordnung der Sensoreinrichtung bzw. des Sensors in der Radialkavität.

Gemäß einem Ausführungsbeispiel ist die Messvorrichtung und/oder das Messsystem eine für den einmaligen Gebrauch vorgesehene Einwegvorrichtung, wobei die Sensoreinrichtung bevorzugt eine Kupplungsstelle für eine Kommunikation und/oder Energieversorgung aufweist, insbesondere für eine drahtgebundene Übertragung über ein Kabel oder für eine drahtlose Übertragung. Die Umspritzung ermöglicht eine einfach aufgebaute Messvorrichtung, bei welcher z.B. lediglich ein Kabel oder ein Stick entfernt werden muss, bevor die Messvorrichtung entsorgt wird. Dabei kann die Messvorrichtung durch mindestens eine der folgenden Maßnahmen in Hinblick auf die Eignung als Einwegvorrichtung optimiert werden: geringer Materialanteil, insbesondere bei der Umspritzung, geringe Anzahl von Bauteilen oder Herstellungsschritte, effizienter Umspritzprozess (insbesondere geringer Energiebedarf).

Die oben angegebene Aufgabe kann auch durch ein medizintechnisches Messsystem mit einer Messvorrichtung zur Messung einer Eigenschaft eines Fluids, insbesondere zur Druckmessung, gelöst werden, bei welchem das Messsystem durch Umspritzen einer Leitung und einer an der Leitung angeordneten Sensoreinrichtung der Messvorrichtung gebildet ist, insbesondere mit einem Schmelzklebstoff. Hierbei ergeben sich die zuvor bereits beschriebenen Vorteile.

Die oben angegebene Aufgabe kann auch durch ein Verfahren zur Herstellung eines medizintechnischen Messsystems gelöst werden. Die Erfindung geht aus von einem Verfahren zur Herstellung eines medizintechnischen Messsystems nach einem der Ansprüche, umfassend die Schritte: Bereitstellen einer Leitung, insbesondere Schlauchleitung, die eingerichtet ist, ein Fluid, insbesondere Blut, zu führen; und Anordnen einer Sensoreinrichtung und wahlweise weiterer Komponenten des Messsystems an der Leitung, insbesondere an einer Außenmantelfläche einer Wandung der Leitung.

Erfindungsgemäß sind zudem die folgenden Verfahrensschritte vorgesehen: Anordnen des Messsystems mit der Leitung in einem (Umspritz-) Werkzeug; und Umspritzen zumindest der Sensoreinrichtung und der Leitung, jeweils zumindest abschnittsweise, insbesondere mit einem Material basierend auf Polyester und Kohlenwasserstoffharz. Hierdurch ergeben sich insbesondere die zuvor in Verbindung mit dem Messsystem beschriebenen Vorteile. Dabei kann das Umspritzmaterial z.B. durch Verflüssigen eines Granulats bereitgestellt werden.

Das Umspritzen erfolgt bevorzugt durch ein Spritzguss-Verfahren, insbesondere durch ein so genanntes "Hotmelt Moulding" oder "Macromelt Moulding" oder durch Niederdruck-Vergusstechnik. Dabei bezieht sich der Begriff "Hotmelt" allgemein auf die Verwendung von Schmelzklebstoffen und umfasst auch solche Schmelzklebstoffe, die als so genannte "Macromelts" bekannt sind.

Das Umspritzen erfolgt bevorzugt durch eine Technik, welche sich zwischen herkömmlichem Spritzguss und einem Zwei-Komponenten-Verguss befindet und sich von diesen Verfahren durch die applizierten Drücken und Zykluszeiten unterscheidet. Der Druck ist bevorzugt kleiner als beim klassischen Spritzguss, und die Zykluszeit ist bevorzugt kleiner als beim Zwei-Komponenten-Verguss. Beim so genannten "Macromelt Moulding" kann ein thermoplastischer Schmelzklebstoff verwendet werden, welcher bevorzugt aus nachwachsenden Rohstoffen besteht. Die Anwendung kann über ein rein thermisches Aufschmelzverfahren ohne chemische Reaktionen erfolgen, ohne dass Schadstoffe frei gesetzt werden müssen.

Beim Umspritzen wird eine Einspritz-Temperatur bevorzugt so gewählt, dass eine Oberfläche (Außenmantelfläche) der Wandung zumindest teilweise aufgeschmolzen wird. Hierdurch kann die Umspritzung auf effektive Weise mit der Wandung verbunden werden, insbesondere durch mechanische Adhäsion. Wahlweise kann dabei auch eine chemische Verbindung zwischen den Komponenten hergestellt werden, sofern erwünscht.

Gemäß einer Ausführungsform wird das Umspritzen zweistufig durchgeführt, indem zunächst in einem ersten Schritt (insbesondere in einem Vor-Verguss-Schritt) zumindest die Sensoreinrichtung umspritzt wird, insbesondere derart, dass die Sensoreinrichtung in einer funktionsfähigen Anordnung bereitgestellt wird, und darauf in einem zweiten Schritt (insbesondere in einem Hauptverguss-Schritt) zumindest auch die Leitung umspritzt wird, wobei die Umspritzung bevorzugt in eine Endform gebracht wird. Hierdurch kann zunächst eine funktionelle Umspritzung derart erfolgen, dass die Sensoreinrichtung funktionsfähig ist, und daraufhin kann die Umspritzung vollständig ausgeformt werden, insbesondere um ein integrales Gehäuse zu bilden und/oder alle Komponenten sicher relativ zueinander zu positionieren und/oder eine große Menge Umspritzmaterial mit großer Geschwindigkeit einzubringen. Durch das zweistufige Verfahren können Einfallstellen vermieden werden, was insbesondere in Hinblick auf eine große Menge Umspritzmaterial Vorteile liefert. Auch kann die erste Umspritzung bei kleinerer Geschwindigkeit und mit größerer Genauigkeit und Dosierung erfolgen, wodurch z.B. eine exaktere Positionierung erfolgen kann, oder wodurch der Umspritzung auf kontrolliertere Weise bestimmte Materialeigenschaften verliehen werden können.

Gemäß einer Ausführungsform wird vor dem Umspritzen ein Dorn in die Leitung eingeführt. Hierdurch kann insbesondere eine Stabilisierung der Leitung sichergestellt werden. Die Umspritzung kann an der Leitung in einem Zustand der Leitung vorgesehen werden, welcher dem Betriebszustand der Leitung entspricht, selbst wenn die Leitung während des Umspritzens einem erhöhten Druck und/oder einer erhöhten Temperatur ausgesetzt wird. Der Dorn kann einen Gegendruck sicherstellen und die Wandung von innen stabilisieren. Auch kann die Wandung mittels des Dorns ausgerichtet und/oder in eine vorbestimmte Geometrie gebracht werden, z.B. auch eine leichte Krümmung. Es hat sich gezeigt, dass über den Durchmesser des Dorns die Verbindung zwischen der Außenmantelfläche der Wandung und dem Umspritzmaterial beeinflusst werden kann. Es hat sich gezeigt, dass ein vergleichsweise großer Dorndurchmesser zu einer besonders guten Anhaftung führt.

Über den Dorndurchmesser kann auch die beim Umspritzen auf die Wandung ausgeübte Kraft eingestellt werden. Auch kann eingestellt werden, in welchem Maße die Wandung einem erhöhten Druck von außen auf die Außenmantelfläche der Wandung nachgeben soll. Insbesondere kann die Leitungs-/Schlauchgeometrie "eingefroren" werden, selbst wenn mit vergleichsweise hohen Drücke im Bereich von 20bar und/oder hohen Temperaturen im Bereich von 180°-200° umspritzt wird. Bevorzugt ist der Dorndurchmesser sowohl in Hinblick auf eine Zuhaltekraft des Umspritzwerkzeugs als auch in Hinblick auf einen Innendurchmesser der Wandung ausgelegt. Mit anderen Worten: Der Durchmesser des Dorns wird bevorzugt derart gewählt, dass ein Optimum an Anpressdruck erzielt wird, so dass die Anhaftung des Umspritzmaterials an der Außenmantelfläche der Wandung maximal ist. Bevorzugt ist der Dorndurchmesser etwas kleiner als der Innendurchmesser der Wandung.

Bevorzugt weist das Umspritzwerkzeug zwei Backen oder Formteile oder auf, welche jeweils einen Teil einer Negativform bereitstellen und aufeinander montierbar sind. Bevorzugt weist das Umspritzwerkzeug hervorstehende Abschnitte zum Ausbilden von Ausnehmungen in der Umspritzung auf, wobei die hervorstehenden Abschnitte bevorzugt eine konkave Oberfläche aufweisen, die jeweils geometrisch korrespondierend zur Außenmantelfläche der Wandung ausgebildet ist.

Gemäß einer Ausführungsform wird vor dem Umspritzen eine Radialkavität zur Aufnahme eines Sensors der Sensoreinrichtung in die Wandung eingebracht und der Sensor in der Radialkavität angeordnet, wobei die Sensoreinrichtung bevorzugt außen an der Wandung fixiert wird, insbesondere stoffschlüssig. Hierdurch kann ein Messsystem mit vorteilhaften Messeigenschaften bereitgestellt werden, und das Positionieren und das Umspritzen der Sensoreinrichtung in der gewünschten (vordefinierten) Position relativ zur Leitung kann vereinfacht werden, insbesondere da die Sensoreinrichtung nicht notwendigerweise in einer bestimmten Position innerhalb eines Werkzeugs gehalten werden muss. Gemäß einer Variante wird die Radialkavität vor dem Umspritzen mittels der Sensoreinrichtung fluiddicht abgedichtet, insbesondere mittels einer Übermaßpassung.

Gemäß einer Ausführungsform wird die Umspritzung derart auf einer Außenmantelfläche der Wandung aufgebracht, dass die Umspritzung an der Außenmantelfläche der Wandung anhaftet, insbesondere durch mechanische Adhäsion, wobei beim Umspritzen bevorzugt eine Temperatur im Bereich von 150° bis 220° und/oder ein Druck im Bereich von 5bar bis 25bar eingestellt wird. Hierdurch kann die Umspritzung zu einer Einheit mit der Wandung verbunden werden, und die Relativposition der Umspritzung relativ zur Leitung kann eindeutig definiert werden, und dadurch auch die Relativposition weiterer Komponenten des Messsystems zueinander. Es hat sich gezeigt, dass eine Anhaftung dabei im Wesentlichen unabhängig von irgendeiner bestimmten Oberflächenbeschaffenheit der Wandung (Struktur, Rauhigkeit) sichergestellt werden kann.

Gemäß einer Ausführungsform wird beim Umspritzen eine Temperatur im Bereich von 150° bis 220° und/oder ein Druck im Bereich von 5bar bis 25bar eingestellt. Hierdurch kann zum einen eine große Menge Umspritzmaterial eingebracht werden, zum anderen kann das Umspritzen derart erfolgen, dass sich die Wandung mit dem Umspritzmaterial verbindet, insbesondere durch physikalische Bindung oder mechanische Adhäsion. Dabei kann das Umspritzverfahren durch weitere Parameter wie einer bestimmten Haltezeit, einer bestimmten Kühlzeit, einer bestimmten Einspritzgeschwindigkeit und/oder einer bestimmten Werkzeug-temperatur definiert werden.

Gemäß einer Ausführungsform werden beim Umspritzen die folgenden Verfahrensparameter eingestellt, wobei auf ein zweistufiges Umspritzverfahren umfassend einen Vor-Verguss-Schritt und einen Hauptverguss-Schritt Bezug genommen wird:

### Tank-Temperatur:

180 bis 200° Celsius (sowohl beim Vor-Verguss als auch beim Hauptverguss);

| | |
|---|---|
| Vorschmelz-Zone: | 180 bis 200° Celsius (Vor-Verguss und Hauptverguss); |
| Leitungs-Temperatur: | 180 bis 200° Celsius (Vor-Verguss und Hauptverguss); |
| Kopf-Temperatur: | 180 bis 200° Celsius (Vor-Verguss und Hauptverguss); |

### Einspritz-Druck:

20bar bis 30bar, insbesondere 25bar (Vor-Verguss und Hauptverguss);

### Haltezeit:

5 bis 15sec (sowohl beim Vor-Verguss als auch beim Hauptverguss);

### Kühlzeit:

50sec bis 70 sec beim Vor-Verguss, 15sec bis 30sec beim Hauptverguss;

| | |
|---|---|
| Massedruck: | 15 bis 20bar beim Vor-Verguss, 20 bis 30bar beim Hauptverguss; |
| Werkzeug-Temperatur: | 45 bis 55° Celsius (Vor-Verguss und Hauptverguss); |

Die genannten Kühlzeiten können eine gute Kohäsion innerhalb der Umspritzung sicherstellen, selbst bei einer vergleichsweise großen Menge Umspritzmaterial. Die gute Kohäsion kann auch eine gute Formbeständigkeit der Umspritzung sicherstellen.

Gemäß einer Ausführungsform erfolgt beim Anordnen im (Umspritz-)Werkzeug ein relatives Positionieren der Sensoreinrichtung relativ zum (Umspritz-)Werkzeug, insbesondere in Bezug auf eine Negativform zum Ausbilden eines Durchlasses in der Umspritzung, z.B. in Bezug auf einen sich in radialer Richtung zumindest annähernd senkrecht/orthogonal zur Mittenlängsachse der Leitung erstreckenden Stift. Hierdurch kann auf einfache Weise bei einer in die Umspritzung eingebetteten Sensoreinrichtung ein Kommunikationspfad zwischen dem Sensor und der Umgebung sichergestellt werden. Die Position der Sensoreinrichtung innerhalb der Umspritzung kann dabei weitgehend beliebig gewählt werden.

Gemäß einer Verfahrensvariante wird ein das Umspritzmaterial aufnehmender Tank ca. auf 170°-180°C temperiert (insbesondere dauerhaft). Die Leitung wird auf ca. 190°-210°, insbesondere 200°C erhitzt (insbesondere kurzzeitig). Eine Düse des Umspritzwerkzeugs wird z.B. auf 200° bis 220°, insbesondere 210°C erhitzt (kurzzeitig). Der im Umspritzwerkzeug eingestellte oder aufgebrachte Druck liegt z.B. zwischen 5bar und 20bar. Das Werkzeug selbst kann ebenfalls temperiert sein, z.B. auf ca. 25° bis 35°, insbesondere 30°C.

Gemäß einer Ausführungsform wird die Leitung beim Anordnen im Werkzeug an mindestens einer Aufnahme des (Umspritz-)Werkzeugs befestigt, insbesondere einem Haltebügel oder einer Halteklammer einer Negativform des (Umspritz-)Werkzeugs. Hierdurch kann die Leitung in einer vorgegebenen SOLL-Geometrie oder Ausrichtung relativ zum Werkzeug und relativ zur Umspritzung angeordnet werden. Bevorzugt ist das Werkzeug zweiteilig aus einem Oberteil und einem Unterteil gebildet.

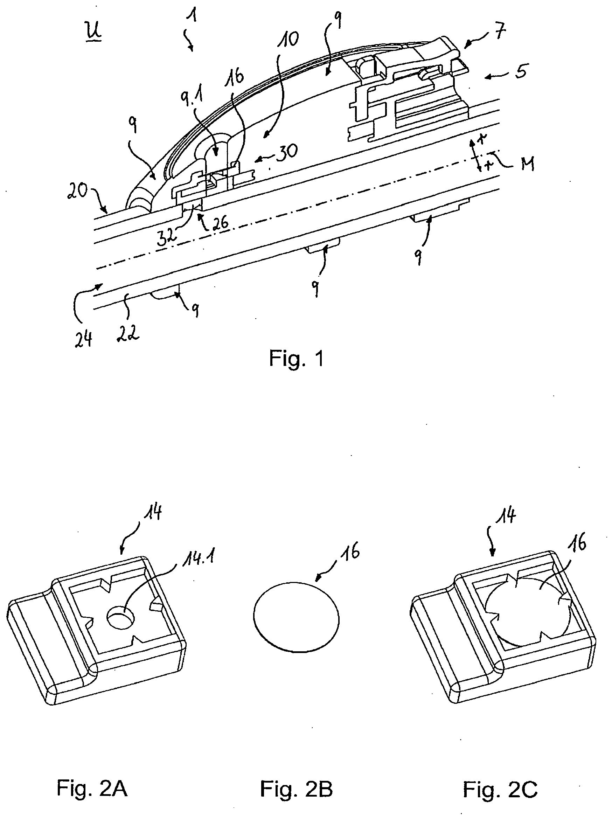

In den nachfolgenden Zeichnungsfiguren wird die Erfindung anhand von Ausführungsbeispielen näher erläutert. Im Zusammenhang mit der Beschreibung wird bei einzelnen Bezugszeichen, sofern diese nicht explizit erläutert werden, auf das Ausführungsbeispiel der Figur 1 oder auf die weiteren Figuren verwiesen. Es zeigen:
Figur 1 in schematischer Darstellung in perspektivischer geschnittener Ansicht ein Messsystem gemäß einem Ausführungsbeispiel der Erfindung;
Figuren 2A, 2B, 2C jeweils in einer perspektivischen Ansicht eine Schutzkappe zum Abdecken einer Sensoreinrichtung des in der Figur 1 gezeigten Messsystems, oder eine Membran für die Schutzkappe;
Figuren 3A, 3B, 3C jeweils in einer perspektivischen Ansicht eine Steckerbuchse zum Verbinden einer Sensoreinrichtung des in der Figur 1 gezeigten Messsystems mit einer Energiequelle und/oder mit Kommunikationsmitteln, z.B. über ein Kabel oder eine drahtlose Verbindung;
Figuren 4A und 4B in einer perspektivischen Ansicht und in einer perspektivischen geschnittenen Ansicht einzelne Komponenten des in der Figur 1 gezeigten Messsystems;
Figur 5 in schematischer Darstellung in perspektivischer geschnittener Ansicht ein Messsystem gemäß einem Ausführungsbeispiel der Erfindung, wobei eine Umspritzung dargestellt ist;
Figuren 6A und 6B jeweils in einer perspektivischen Ansicht ein Messsystem gemäß einem Ausführungsbeispiel der Erfindung;
Figuren 7A und 7B jeweils in einer perspektivischen Ansicht ein Messsystem gemäß einem weiteren Ausführungsbeispiel der Erfindung; und
Figur 8 in schematischer Darstellung ein Verfahrensschaubild zu Schritten eines Verfahrens gemäß Ausführungsbeispielen der Erfindung.
Figur 9 In schematischer Darstellung in Aufsicht auf die Unterseite ein Messsystem gemäß einem Ausführungsbeispiel der Erfindung, wobei eine Umspritzung dargestellt ist.

In der Figur 1 ist ein medizintechnisches Messsystem 1 umfassend eine sich in Längsrichtung entlang einer Mittenlängsachse M erstreckende Leitung 20 und eine Messvorrichtung 10 mit einer Sensoreinrichtung 30 gezeigt. Die Leitung 20 ist in der Figur 1 am Beispiel einer Schlauchleitung erläutert. Die Sensoreinrichtung 30 weist einen Sensor 32 auf, welcher in einer Radialkavität 26 innerhalb einer Wandung 22 der Leitung 20 angeordnet ist. Die Radialkavität 26 erstreckt sich in radialer Richtung r und ist in Form einer Öffnung, eines Lochs oder einer Bohrung in der Wandung 22 ausgebildet. Der Sensor 32 ist eingerichtet, eine Eigenschaft, insbesondere einen Druck eines in einer Längskavität 24 innerhalb der Leitung 20 geführten Fluids zu messen. Das Messsystem 1 umfasst ferner eine Steckerbuchse 5 und einen Taster 7.

Das Messsystem 1 weist eine Umspritzung 9 auf, welche die Sensoreinrichtung 30 und zumindest teilweise auch die Steckerbuchse 5 und den Taster 7 umgibt. Die Umspritzung 9 ist zumindest abschnittsweise in Kontakt mit der Wandung 22. Die Umspritzung 9 umringt die Wandung 22 zumindest abschnittsweise, insbesondere an drei unterschiedlichen Stellen, nämlich ganz vorne, etwa mittig, und ganz hinten von der gezeigten Anordnung. Die Umspritzung 9 weist einen Durchlass 9.1 oder eine Öffnung auf, über welche der Sensor 32 in Verbindung mit der Umgebung U steht. Der Durchlass 9.1 ist im Bereich einer Abdeckung/Schutzkappe 14, insbesondere im Bereich einer Membran 16 der Sensoreinrichtung 30 angeordnet und erstreckt sich im Wesentlichen in radialer Richtung.

In den Figuren 2A, 2B und 2C sind die Komponenten Schutzkappe 14 und Membran 16 im Detail gezeigt. Die Schutzkappe 14 weist eine Öffnung 14.1 auf, insbesondere in Form einer Entlüftungsbohrung. Die bevorzugt gaspermeable, fluiddichte Membran 16 ist über der Öffnung 14.1 angeordnet und bedeckt diese vollständig. Die Membran 16 kann dabei an einem Außenrand an mehreren Punkten befestigt sein, insbesondere an in radialer Richtung nach innen hervorstehenden Nasen oder Haken.

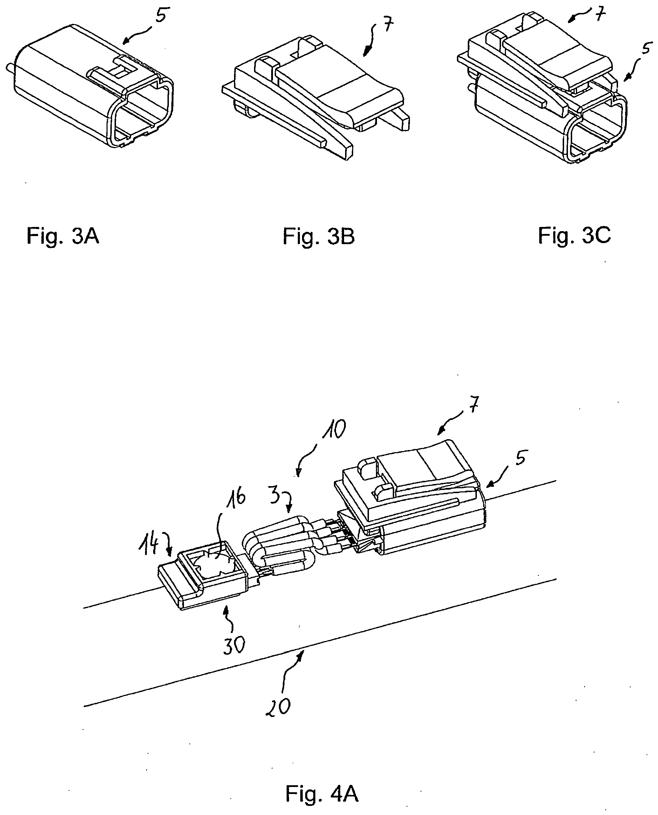

In den Figuren 3A, 3B und 3C sind die Komponenten Steckerbuchse 5 und Taster 7 im Detail gezeigt. Der Taster 7 kann auf der Steckerbuchse 5 angeordnet werden. Eine Unterseite des Tasters 7 ist geometrisch korrespondierend zu einer Oberseite der Steckerbuchse 5 ausgebildet.

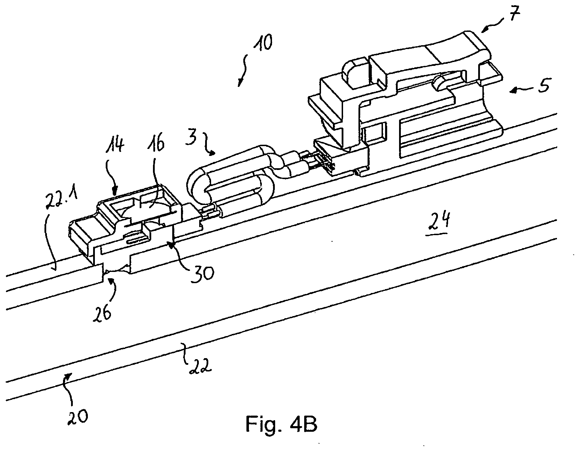

In den Figuren 4A und 4B ist noch eine weitere Komponente der Messvorrichtung 10 gezeigt. Die Messvorrichtung 10 umfasst auch ein Adapterkabel 3, welches zwischen der Steckerbuchse 5 und der Sensoreinrichtung 30 angeordnet ist und die Sensoreinrichtung 30 elektrisch mit einer Energieversorgung und/oder Kommunikationsschnittstelle (nicht gezeigt) verbindet. Das Adapterkabel 3 ist in Schleifen oder Windungen oder schlangenartig mit Überlänge zwischen der Steckerbuchse 5 und der Sensoreinrichtung 30 angeordnet, wodurch Relativbewegungen, z.B. aufgrund einer Biegung der Leitung 20, ausgeglichen werden können.

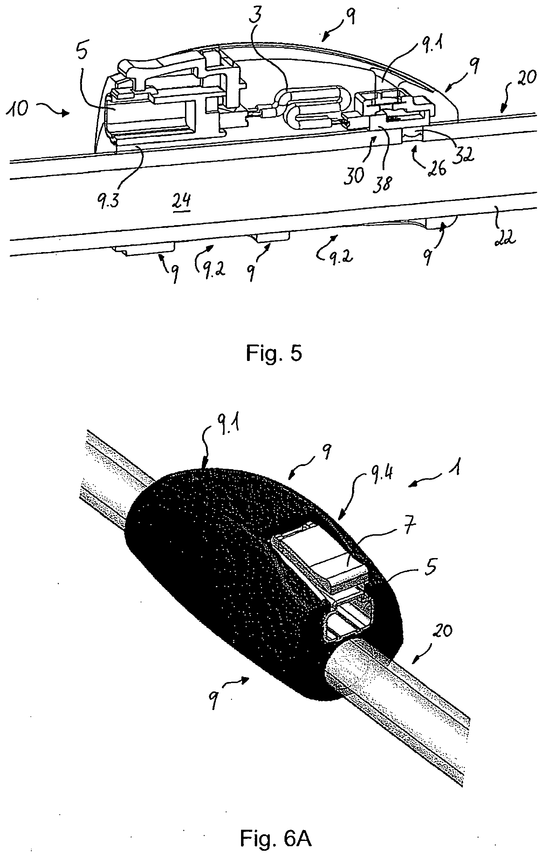

In der Figur 4B ist im Detail gezeigt, dass die Sensoreinrichtung 30 in Kontakt mit einer Außenmantelfläche 22.1 der Leitung 22 ist, wohingegen das Adapterkabel 3 und die Steckerbuchse 5 in die Umspritzung eingebettet sind, wie in Figur 5 gezeigt.

In der Figur 5 ist die Umspritzung 9 im Querschnitt gezeigt. Das Adapterkabel 3 und die Steckerbuchse 5 sind in die Umspritzung 9 eingebettet und berühren die Leitung 20 nicht. Eine Reibung dieser Komponenten an der Wandung 22 kann dadurch vermieden werden. Zwischen der Steckerbuchse 5 und der Wandung 22 ist ein Zwischenabschnitt 9.3 ausgebildet, welcher die Steckerbuchse 5 relativ zur Wandung 22 positioniert. Im Gegensatz dazu weist die Sensoreinrichtung 30 einen Anlageabschnitt 38 auf, welcher außen an der Wandung 22 angeordnet ist und bevorzugt stoffschlüssig mit der Wandung 22 verbunden ist. Auf diese Weise kann die Radialposition des Sensors 32 innerhalb der Radialkavität 26 auf exakte Weise definiert werden, und eine Relativbewegung zwischen der Wandung 22 und der Sensoreinrichtung 30 kann vermieden werden. Dies kann eine gute Fluiddichtigkeit und eine eindeutige Position des Sensors 32 und damit eine exakte Messung sicherstellen. Gleichzeitig kann die Umspritzung 9 sicherstellen, dass keine Radial- oder Axialkräfte von extern auf die Sensoreinrichtung 30 ausgeübt werden. Die Umspritzung 9 kann die Sensoreinrichtung 30 vor externen Einflüssen schützen und die vordefinierte Position der Sensoreinrichtung 30 relativ zur Wandung 22 sicherstellen durch Verhindern von Relativbewegungen zwischen der Sensoreinrichtung 30 und der Wandung 22.

An einer Unterseite der Wandung 22 weist die Umspritzung 9 zwei Aussparungen 9.2 auf. Diese Aussparungen 9.2 können einerseits den Materialverbrauch gering halten, andererseits kann eine Flexibilität der Leitung 20 aufrechterhalten werden, oder aufgrund von Verformungen in der Umspritzung hervorgerufene Spannungen können gering gehalten werden.

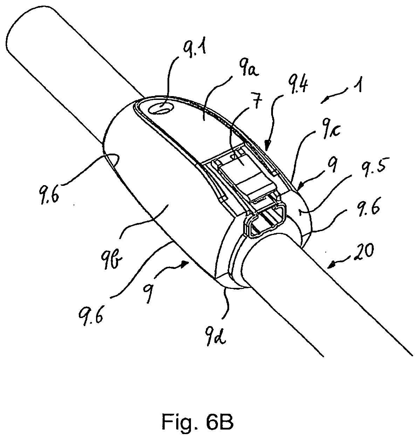

In den Figuren 6A und 6B ist die Umspritzung 9 von einer Ansicht auf eine hintere, proximale Seite gezeigt, an welcher die Steckerbuchse 5 mit einem Kabel oder einem (Kommunikations-)Stick verbunden werden kann. Die in den Figuren gezeigte Steckerbuchse 5 ist eingerichtet, entweder einen Stecker in Verbindung mit einem (externen) Kabel oder eine Art "Stick" oder Modul aufzunehmen. Der Stick kann eine drahtlose Kommunikation sicherstellen, z.B. über WLAN, Funk, bluetooth. Der Stick kann auch eine Energieversorgung aufweisen, z.B. einen Akku. Die Steckerbuchse 5 kann dabei für beide Varianten dieselbe Form aufweisen, so dass ein Benutzer jeweils entscheiden kann, ob eine drahtgebundene Energieversorgung und Kommunikation erwünscht ist, oder ob die Kommunikation drahtlos und die Energieversorgung über den Stick, z.B. mittels im Stick integrierten Batterien, erfolgen soll. Sowohl das Kabel als auch der Stick können dabei mehrfach verwendet werden. Mit anderen Worten: das medizintechnische Messsystem 1 oder die medizintechnische Messvorrichtung 10 können für den Einmalgebrauch ("disposable") vorgesehen sein, und vor dem Entsorgen kann das Kabel oder der Stick dann von der Steckerbuchse entkoppelt werden.

Die Umspritzung 9 umgibt die Leitung 20 vollumfänglich. An einer Oberseite weist die Umspritzung 9 eine Ausnehmung oder Aussparung 9.4 zur Aufnahme des Tasters 7 auf. Ein proximaler Stirnflächenabschnitt 9.5 der Umspritzung 9 ist in derselben Radialebene angeordnet wie ein proximales Ende der Steckerbuchse 5. Die Umspritzung 9 und die Steckerbuchse 5 sind bündig in derselben Ebene angeordnet. Dies kann eine kompakte Anordnung sicherstellen, und Kanten oder Vorsprünge können in die Umspritzung 9 integriert werden, so dass ein Benutzer nicht in Kontakt mit den Kanten kommt.

Die Umspritzung 9 weist mehrere Flächenabschnitte auf, insbesondere vier unterschiedliche Flächenabschnitte. Ein oberseitiger Flächenabschnitt 9a liefert eine Abdeckung und den Durchlass 9.1. Seitliche (laterale) Flächenabschnitte 9b, 9c liefern Flanken oder Halteflächen, an welchen die Umspritzung 9 auf ergonomische Weise ergriffen und gehalten werden kann. Ein unterseitiger Flächenabschnitt 9d liefert eine Rundung und eine Unterseite, auf welcher die Umspritzung 9 abgelegt werden kann, z.B. beim Verbinden der Steckerbuchse 5 mit einem Kabel. Der jeweilige seitliche (laterale) Flächenabschnitt 9b, 9c grenzt an einer Kante 9.6 an den unteren Flächenabschnitt 9d an. Die Kante 9.6 erstreckt sich in Längsrichtung entlang der gesamten Umspritzung 9 und kann eine ergonomische Negativform für die Innenfläche einer menschlichen Hand bereitstellen. Im Querschnitt ist die Umspritzung 9 rautenartig mit konvex-runden Flächenabschnitten ausgebildet.

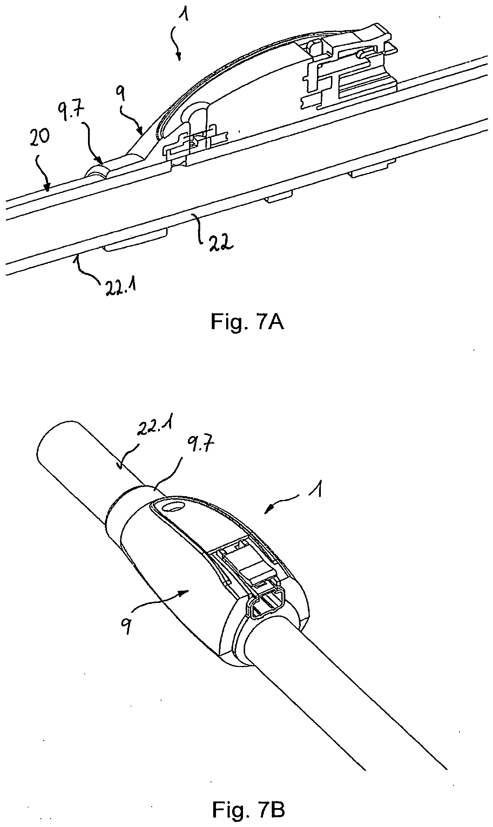

In den Figuren 7A und 7B ist ein Ausführungsbeispiel gezeigt, bei welchem die Umspritzung 9 einen Fortsatz 9.7 aufweist. Der Fortsatz 9.7 ist an einem distalen Ende der Umspritzung vorgesehen und kommt an einer/der Außenmantelfläche 22.1 der Wandung 22 zur Anlage. Wahlweise kann der Fortsatz auch an einem proximalen Ende der Umspritzung vorgesehen sein, auch zusätzlich.

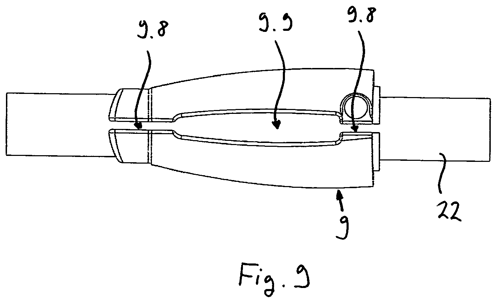

In Figur 9 ist ein Ausführungsbeispiel gezeigt, bei welchem die Umspritzung 9 an einer Unterseite der Wandung 22 einen parallel zur Längserstreckung der Wandung 22 verlaufenden durchgehenden Schlitz 9.8 aufweist. Dieser Schlitz 9.8 ist in einem zentralen Bereich zu einem Fenster 9.9 verbreitert, wodurch in diesem Bereich die Wandung 22 durchgängig sichtbar ist. Ist die Wandung 22 aus einem transparenten Material gefertigt, dann wird ein durchgängiger Blick auf den Sensor 32 in der Radialkavität 26 bzw. auf ein in der Leitung 20 geführtes Fluid möglich. Ferner kann der Schlitz 9.8 in Längsrichtung auch als Dehnfuge fungieren, die die Gefahr einer bewegungsbedingten Rissbildung ausschließt.

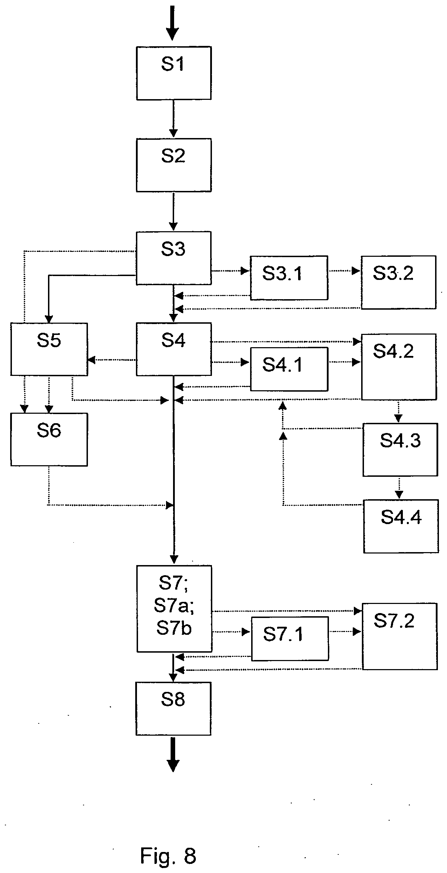

In der Figur 8 sind Schritte eines Verfahrens zur Herstellung eines medizintechnischen Messsystems dargestellt. Das Verfahren umfasst einen oder mehrere der im Folgenden beschriebenen Schritte.

In einem Schritt S1 erfolgt ein Bereitstellen einer Leitung, die eingerichtet ist, ein Fluid, insbesondere Blut, zu führen. In einem Schritt S2 erfolgt ein Einbringen einer Radialkavität in eine Wandung der Leitung, beispielsweise durch Bohren. In einem Schritt S3 erfolgt ein Anordnen einer Sensoreinrichtung und wahlweise weiterer Komponenten des Messsystems an der Leitung, insbesondere an einer Außenmantelfläche der Wandung. Dabei kann die Messeinrichtung in einem Schritt S3.1 an der Wandung fixiert werden, insbesondere aus stoffschlüssige Weise mittels eines Haftmittels. Ferner kann die Radialkavität in einem Schritt S3.2 abgedichtet werden, insbesondere indem ein Radialabschnitt der Sensoreinrichtung eine Übermaßpassung mit der Radialkavität bildet. Ein Haftmittel ist dabei bevorzugt nicht vorgesehen. Die Schritte S3.1 und S3.2 können unabhängig voneinander oder in Verbindung miteinander durchgeführt werden. In einem darauffolgenden Schritt S4 kann das Messsystem zusammen mit der Leitung in einem Umspritz-Werkzeug angeordnet werden. Dabei kann in einem Schritt S4.1 ein relatives Positionieren der Sensoreinrichtung im Umspritz-Werkzeug erfolgen, insbesondere in Bezug auf einen hervorstehenden (insbesondere radial ausgerichteten) Stift oder in Verbindung mit einem Anordnen des Stiftes zum Ausbilden eines Durchlasses in der Umspritzung. Zusätzlich kann auch in einem Schritt S4.2 mindestens eine weitere Komponente des Messsystems, insbesondere eine Steckerbuchse und/oder ein Adapterkabel, im Umspritz-Werkzeug angeordnet werden, und in einem Schritt S4.3 relativ zum Umspritz-Werkzeug positioniert werden, und/oder in einem Schritt S4.4 kann die Steckerbuchse relativ zur Außenmantelfläche der Wandung positioniert werden.

Die Steckerbuchse des Messsystems kann beim Anordnen an der Leitung relativ zur Leitung positioniert werden, wahlweise in Verbindung mit einem Taster und/oder einem Adapterkabel. Die Steckerbuchse kann dabei in einem Radialabstand beabstandet zu einer Außenmantelfläche der Wandung positioniert werden. Bevorzugt beträgt der Radialabstand mindestens 1.0mm. Es hat sich gezeigt, dass dieser Abstand ausreichend groß ist, damit das Umspritzmaterial in den Zwischenraum zwischen die Wandung und die Steckerbuchse eingebracht werden kann.

In einem Schritt S5 können die einzelnen Komponenten miteinander verbunden werden. Insbesondere kann die Sensoreinrichtung mit einem Adapterkabel verbunden werden, und das Adapterkabel kann mit der Steckerbuchse verbunden werden. Der Schritt S5 kann dabei auch vor dem Schritt S4 durchgeführt werden.

Nachdem die Anordnung des Messsystems im Werkzeug erfolgt ist und das Werkzeug geschlossen wurde, kann in einem Schritt S7 ein Umspritzen erfolgen. Der Schritt S7 kann dabei durch die folgenden Schritte gekennzeichent sein: Schritt S7a, entsprechend einem Verfahrensschritt einer Vor-Umspritzung (erster Umspritzungsschritt), Schritt S7b, entsprechend einem Verfahrensschritt einer Haupt-Umspritzung (zweiter Umspritzungsschritt), Schritt S7.1, entsprechend einem Verfahrensschritt des Einstellens einer spezifischen Temperatur beim Umspritzen, Schritt S7.2, entsprechend einem Verfahrensschritt des Einstellens eines spezifischen Drucks beim Umspritzen. Wahlweise kann vor dem Umspritzen, insbesondere auch vor dem Schritt S4, in einem Schritt S6 ein Anordnen eines Dorns in einer Längskavität der Leitung erfolgen. Das Umspritzen kann dabei durch Einstellen einer spezifischen Temperatur (Schritt 7.1) und/oder durch ein Einstellen eines spezifischen Drucks (Schritt 7.2) gesteuert werden. Schließlich kann in einem Schritt S8 ein Entformen erfolgen, insbesondere nach einer vorbestimmten Abkühlzeit.

### Bezugszeichenliste

- 1: medizintechnisches Messsystem
- 3: Adapterkabel
- 5: Steckerbuchse
- 7: Taster
- 9: Umspritzung
- 9a: oberseitiger Flächenabschnitt
- 9b, 9c: seitlicher (lateraler) Flächenabschnitt
- 9d: unterseitiger Flächenabschnitt
- 9.1: Durchlass oder Öffnung in Umspritzung
- 9.2: Aussparung an Unterseite
- 9.3: Zwischenabschnitt
- 9.4: Ausnehmung oder Aussparung an Oberseite
- 9.5: proximale Stirnfläche oder Stirnflächenabschnitt
- 9.6: Kante
- 9.7: Fortsatz oder Rand
- 9.8: Schlitz
- 9.9: Fenster an Unterseite

- 10: medizintechnische Messvorrichtung
- 14: Abdeckung, insbesondere Schutzkappe
- 14.1: Öffnung, insbesondere Entlüftungsbohrung
- 16: gaspermeable, fluiddichte Membran

- 20: Leitung, insbesondere Schlauchleitung
- 22: Wandung
- 22.1: Außenmantelfläche der Wandung
- 24: Längskavität entlang der Mittenlängsachse für Führung des Fluids
- 26: Radialkavität, insbesondere Öffnung oder Ausnehmung

- 30: Sensoreinrichtung
- 32: Sensor
- 38: Anlageabschnitt

- M: Mittenlängsachse
- r: radiale Richtung

- S1: Verfahrensschritt des Bereitstellens einer Leitung
- S2: Verfahrensschritt des Einbringens einer Radialkavität
- S3: Verfahrensschritt des Anordnens einer Sensoreinrichtung an der Leitung
- S3.1: Verfahrensschritt des Fixierens der Sensoreinrichtung an der Leitung
- S3.2: Verfahrensschritt des Abdichtens der Radialkavität mittels der Sensoreinrichtung
- S4: Verfahrensschritt des Anordnens des Messsystems mit der Leitung in einem Umspritz-Werkzeug
- S4.1: Verfahrensschritt des relativen Positionierens der Sensoreinrichtung im Umspritz-Werkzeug
- S4.2: Verfahrensschritt des Anordnens mindestens einer weiteren Komponente des Mess-systems im Umspritz-Werkzeug
- S4.3: Verfahrensschritt des relativen Positionierens der Steckerbuchse im Umspritz-Werkzeug
- S4.4: Verfahrensschritt des relativen Positionierens der Steckerbuchse relativ zur Außenmantelfläche der Wandung
- S5: Verfahrensschritt des Verbindens, insbesondere elektrischen Verbindens einzelner Komponenten des Messsystems miteinander
- S6: Verfahrensschritt des Anordnens eines Dorns in der Längskavität der Leitung
- S7: Verfahrensschritt des Umspritzens
- S7a: Verfahrensschritt einer Vor-Umspritzung (erster Umspritzungsschritt)
- S7b: Verfahrensschritt einer Haupt-Umspritzung (zweiter Umspritzungsschritt)
- S7.1: Verfahrensschritt des Einstellens einer spezifischen Temperatur beim Umspritzen
- S7.2: Verfahrensschritt des Einstellens eines spezifischen Drucks beim Umspritzen
- S8: Verfahrensschritt des Entformens

- U: Umgebung

## Patentansprüche

1. Medizintechnisches Messsystem (1) mit einer Messvorrichtung (10) zur Messung einer Eigenschaft eines Fluids, insbesondere zur Druckmessung, wobei die Messvorrichtung umfasst:
- eine Leitung (20), die sich entlang einer Mittenlängsachse (M) erstreckt und eingerichtet ist, ein Fluid, insbesondere Blut, innerhalb einer von einer Wandung umgrenzten Längskavität (24) zu führen;
- eine Sensoreinrichtung (30) mit einem Sensor (32), die eingerichtet ist, eine Eigenschaft des in der Längskavität geführten Fluids zu messen;
wobei das Messsystem (1) eine Umspritzung (9) aufweist, welche die Messvorrichtung (10) zumindest teilweise umgibt und wobei die Umspritzung (9) sowohl zumindest die Sensoreinrichtung (30) als auch einen Abschnitt der Leitung (20) umgibt, **dadurch gekennzeichnet, dass**
das Messsystem (1) ein mit der Sensoreinrichtung (30) verbundenes Adapterkabel (3) aufweist, welches in die Umspritzung (9) eingebettet ist.

2. Messsystem (1) nach Anspruch 1, wobei die Umspritzung (9) derart an der Leitung (20) vorgesehen ist, dass die Umspritzung (9) zumindest die Sensoreinrichtung (30) oder auch zusätzliche Komponenten der Messvorrichtung (10) umgibt, wobei zumindest die Sensoreinrichtung (30) in einer vordefinierten Position relativ zur Leitung (20) in der Umspritzung (9) positioniert ist, insbesondere eingebettet ist.

3. Messsystem (1) nach einem der vorhergehenden Ansprüche, wobei die Umspritzung (9) aus einem Schmelzklebstoff gebildet ist.

4. Messsystem (1) nach Anspruch 3, wobei die Umspritzung (9) aus einer Mischung aus Polyester und Kohlenwasserstoffharz ausgebildet ist, insbesondere mit einem größeren Anteil Polyester als Kohlenwasserstoffharz.

5. Messsystem (1) nach einem der vorhergehenden Ansprüche, wobei die Umspritzung (9) eingerichtet ist, an einer Außenmantelfläche (22.1) der Wandung (22) anzuhaften, insbesondere durch mechanische Adhäsion.

6. Messsystem (1) nach einem der vorhergehenden Ansprüche, wobei die Umspritzung (9) zumindest die Leitung (20) zumindest abschnittsweise vollumfänglich umgibt, insbesondere an mindestens zwei voneinander beabstandeten Abschnitten der Umspritzung (9), bevorzugt an zwei in maximalem Abstand zueinander angeordneten freien Enden der Umspritzung (9).

7. Messsystem (1) nach einem der vorhergehenden Ansprüche, wobei die Umspritzung (9) an einem proximalen und/oder distalen Ende oder Abschnitt der Umspritzung einen Fortsatz oder Rand (9.7) aufweist, welcher an einer/der Außenmantelfläche (22.1) der Wandung zur Anlage kommt.

8. Messsystem (1) nach einem der vorhergehenden Ansprüche, wobei die Umspritzung (9) einen sich bevorzugt in radialer Richtung, insbesondere zumindest annähernd senkrecht/orthogonal zur Mittenlängsachse (M) erstreckenden Durchlass (9.1) aufweist, wobei der Durchlass eine Verbindung zwischen dem Sensor (32) und der Umgebung (U) bereitstellt.

9. Messsystem (1) nach einem der vorhergehenden Ansprüche, wobei das Messsystem (1) eine Steckerbuchse (5) aufweist, wobei die Umspritzung (9) die Steckerbuchse zumindest teilweise umgibt und bevorzugt auch zwischen der Leitung (20) und der Steckerbuchse vorgesehen ist.

10. Messsystem (1) nach einem der vorhergehenden Ansprüche, wobei das Adapterkabel mit Überlänge, insbesondere in einer Schlangenlinie, in der Umspritzung (9) angeordnet ist.

11. Messsystem (1) nach einem der vorhergehenden Ansprüche, wobei die Leitung (20) eine in radialer Richtung in die Wandung eingebrachte Radialkavität (26) aufweist, in welcher zumindest der Sensor (32) der Sensoreinrichtung (30) angeordnet ist und derart in die Wandung integriert ist, dass der Sensor (32) in Kommunikation mit dem in der Längskavität (24) geführten Fluid ist.

12. Messsystem (1) nach einem der vorhergehenden Ansprüche, wobei die Messvorrichtung (10) und/oder das Messsystem (1) eine für den einmaligen Gebrauch vorgesehene Einwegvorrichtung ist, wobei die Sensoreinrichtung (30) bevorzugt eine Kupplungsstelle für eine Kommunikation und/oder Energieversorgung aufweist, insbesondere für eine drahtgebundene Übertragung über ein Kabel oder für eine drahtlose Übertragung.

13. Verfahren zur Herstellung eines medizintechnischen Messsystems nach einem der vorhergehenden Ansprüche 1 bis 12, umfassend die Schritte:
- Bereitstellen einer Leitung (20), die eingerichtet ist, ein Fluid, insbesondere Blut, zu führen;
- Anordnen einer Sensoreinrichtung (30) und wahlweise weiterer Komponenten des Messsystems an der Leitung (20), insbesondere an einer Außenmantelfläche (22.1) einer Wandung der Leitung (20);
- Anordnen des Messsystems mit der Leitung (20) in einem Werkzeug;
- Umspritzen zumindest der Sensoreinrichtung (30) und der Leitung (20), jeweils zumindest abschnittsweise, insbesondere mit einem Material basierend auf Polyester und Kohlenwasserstoffharz.

14. Verfahren nach Anspruch 13, wobei das Umspritzen zweistufig durchgeführt wird, indem zunächst in einem ersten Schritt zumindest die Sensoreinrichtung (30) umspritzt wird, insbesondere derart, dass die Sensoreinrichtung (30) in einer funktionsfähigen Anordnung bereitgestellt wird, und darauf in einem zweiten Schritt zumindest auch die Leitung (20) umspritzt wird, wobei die Umspritzung (9) bevorzugt in eine Endform gebracht wird, wobei vor dem Umspritzen bevorzugt ein Dorn in die Leitung (20) eingeführt wird.

15. Verfahren nach einem der vorhergehenden Verfahrensansprüche, wobei beim Anordnen im Werkzeug ein relatives Positionieren der Sensoreinrichtung (30) relativ zum Werkzeug erfolgt, insbesondere in Bezug auf eine Negativform zum Ausbilden eines Durchlasses in der Umspritzung (9), wobei die Leitung (20) beim Anordnen im Werkzeug bevorzugt an mindestens einer Aufnahme des Werkzeugs befestigt wird, insbesondere an einem Haltebügel oder einer Halteklammer einer Negativform des Werkzeugs.

## Claims

1. Medical technology measuring system (1) with a measuring device (10) for measuring a characteristic of a fluid - in particular, for pressure measurement - wherein the measuring device comprises:
- a lead (20), which extends along a central longitudinal axis (M) and is configured to guide a fluid - in particular, blood - within a longitudinal cavity (24) bounded by a wall (24);
- a sensor device (30) with a sensor (32), which is configured to measure a characteristic of the fluid guided in the longitudinal cavity;
wherein the measuring system (1) has an overmolding (9), which at least partially surrounds the measuring device (10) and wherein the overmolding (9) surrounds both at least the sensor device (30) as well as a section of the lead (20), **characterized in that**
the measuring system (1) has an adapter cable (3) connected to the sensor device (30), which is embedded in the overmolding (9).

2. Measuring system (1) according to claim 1, wherein the overmolding (9) is provided on the lead (20) such that the overmolding (9) surrounds at least the sensor device (30) or also additional components of the measuring device (10), wherein at least the sensor device (30) is positioned - in particular, embedded - in a predefined position relative to the lead (20) in the overmolding (9).

3. Measuring system (1) according to one of the preceding claims, wherein the overmolding (9) is made of a hotmelt adhesive.

4. Measuring system (1) according to claim 3, wherein the overmolding (9) is made of a mixture of polyester and hydrocarbon resin - in particular, with a greater part polyester than hydrocarbon resin.

5. Measuring system (1) according to one of the preceding claims, wherein the overmolding (9) is configured to adhere on an outer lateral surface (22.1) of the wall (22) - in particular, by mechanical adhesion.

6. Measuring system (1) according to one of the preceding claims, wherein the overmolding (9) completely surrounds at least the lead (20), at least in sections - in particular, on at least two sections of the overmolding (9) spaced apart from one another, and preferably on two free ends of the overmolding (9) arranged at a maximum distance from one another.

7. Measuring system (1) according to one of the preceding claims, wherein the overmolding (9) has an extension or edge (9.7) on a proximal and/or distal end or section of the overmolding, which comes into contact on an/the outer lateral surface (22.1) of the wall.

8. Measuring system (1) according to one of the preceding claims, wherein the overmolding (9) has an opening (9.1) extending preferably in a radial direction
- in particular, at least approximately vertically/orthogonally to the central longitudinal axis (M), wherein the opening provides a connection between the sensor (32) and the surroundings (U).

9. Measuring system (1) according to one of the preceding claims, wherein the measuring system (1) has a receptacle (5), wherein the overmolding (9) at least partially surrounds the receptacle and preferably is also provided between the lead (20) and the receptacle.

10. Measuring system (1) according to one of the preceding claims, wherein the adapter cable is arranged with excess length - in particular, in a serpentine line - in the overmolding (9).

11. Measuring system (1) according to one of the preceding claims, wherein the lead (20) has a radial cavity (26) incorporated in the wall in a radial direction, in which at least the sensor (32) of the sensor device (30) is arranged and is integrated in the wall such that the sensor (32) is in communication with the fluid guided in the longitudinal cavity (24).

12. Measuring system (1) according to one of the preceding claims, wherein the measuring device (10) and/or the measuring system (1) is a disposable device provided for single use, wherein the sensor device (30) preferably has a coupling point for communication and/or power supply - in particular, for a wired transmission via a cable or for a wireless transmission.

13. Method for producing a medical technology measuring system (1) according to any one of the preceding claims 1 to 12 comprising the steps:
- provision of a lead (20), that is configured to guide a fluid - in particular, blood;
- arrangement of a sensor device (30) and, optionally, further components of the measuring system on the lead (20) - in particular, an outer lateral surface (22.1) of a wall of the lead (20);
- arrangement of the measuring system with the lead (20) in a tool;
- overmolding at least the sensor device (30) and the lead (20) - in each case, at least in sections - in particular, with a material based upon polyester and hydrocarbon resin.

14. Method according to claim 13, wherein the overmolding is carried out in two steps, by means of, in a first step, overmolding at least the sensor device (30)
- in particular, such that the sensor device (30) is provided in a functional arrangement - and then, in a second step, overmolding at least also the lead (20), wherein the overmolding (9) is preferably brought into a final form, wherein, prior to overmolding, preferably a mandrel is inserted into the lead (20).

15. Method according to one of the preceding method claims, wherein, in the case of arrangement in the tool, a relative positioning of the sensor device (30) relative to the tool takes place - in particular, in relation to a negative mold for the formation of an opening in the overmolding (9), wherein the lead (20), in the case of arrangement in the tool, is preferably fastened on at least one holding fixture of the tool, in particular, on a retaining bracket or a retaining clip of a negative mold of the tool.

## Revendications

1. Système de mesure médico-technique (1) comprenant un dispositif de mesure (10) servant à mesurer une propriété d'un fluide, en particulier sa pression, dans lequel le dispositif de mesure comprend :
- une conduite (20) qui s'étend le long d'un axe médian longitudinal (M) et qui est adaptée pour guider un fluide, en particulier du sang, à l'intérieur d'une cavité longitudinale (24) délimitée par une paroi,
- un dispositif de détection (30) comprenant un capteur (32) qui est adapté pour mesurer une propriété du liquide guidé dans la cavité longitud inale,
dans lequel le système de mesure (1) comprend un surmoulage (9) qui entoure au moins en partie le dispositif de mesure (10) et le surmoulage (9) entoure au moins le système de détection (30) ainsi qu'une partie de la conduite (20),
**caractérisé en ce que** le système de mesure (1) comprend un câble adaptateur (3) relié au dispositif de détection (30) qui est encastré dans le surmoulage (9).

2. Système de mesure (1) selon la revendication 1, dans lequel le surmoulage (9) est agencé sur la conduite (20) de telle manière que le surmoulage (9) entoure au moins le dispositif de détection (30) ou aussi des composantes supplémentaires du dispositif de mesure (10), dans lequel au moins le dispositif de détection (30) est positionné, en particulier encastré dans le surmoulage (9) dans une position prédéfinie par rapport à la conduite (20).

3. Système de mesure (1) selon l'une des revendications précédentes, dans lequel le surmoulage (9) est constitué d'un adhésif thermofusible.

4. Système de mesure (1) selon la revendication 3, dans lequel le surmoulage (9) est formé d'un mélange de polyester et de résine hydrocarbonée, en particulier qui contient une plus grande proportion de polyester que de résine hydrocarbonée.

5. Système de mesure (1) selon l'une des revendications précédentes, dans lequel le surmoulage (9) est adapté pour adhérer à une surface périphérique extérieure (22.1) de la paroi (22), notamment par adhésion mécanique.

6. Système de mesure (1) selon l'une des revendications précédentes, dans lequel le surmoulage (9) entoure au moins la conduite (20) intégralement au moins sur une partie, en particulier sur au moins deux parties du surmoulage (9) espacées l'une de l'autre, de préférence sur deux extrémités libres du surmoulage (9) disposées à une distance maximale l'une de l'autre.

7. Système de mesure (1) selon l'une des revendications précédentes, dans lequel le surmoulage (9) comprend un prolongement ou un bord (9.7) à une extrémité ou partie proximale et/ou distale du surmoulage, lequel vient en appui contre une / la surface périphérique extérieure (22.1) de la paroi

8. Système de mesure (1) selon l'une des revendications précédentes, dans lequel le surmoulage (9) comprend un passage (9.1) s'étendant de préférence dans la direction radiale, en particulier au moins approximativement perpendiculairement / orthogonalement par rapport à l'axe longitudinal médian (M), dans lequel le passage fournit une jonction entre le capteur (32) et la zone environnante (U).

9. Système de mesure (1) selon l'une des revendications précédentes, dans lequel le système de mesure (1) comprend un connecteur femelle (5), dans lequel le surmoulage (9) entoure au moins en partie le connecteur femelle et est disposé en outre de préférence entre la conduite (20) et le connecteur femelle.

10. Système de mesure (1) selon l'une des revendications précédentes, dans lequel le câble adaptateur est disposé avec une surlongueur, en particulier dans un tracé sinueux, dans le surmoulage (9).

11. Système de mesure (1) selon l'une des revendications précédentes, dans lequel la conduite (20) comprend une cavité radiale (26) disposée dans la direction radiale dans la paroi, dans laquelle au moins le capteur (32) du dispositif de détection (30) est disposé et est intégré dans la paroi de telle manière que le capteur est en communication avec le fluide guidé dans la cavité allongée (24).

12. Système de mesure (1) selon l'une des revendications précédentes, dans lequel le dispositif de mesure (10) et/ou le système de mesure (1) est un dispositif jetable destiné à un usage unique, dans lequel le dispositif de détection (30) comprend de préférence un emplacement de couplage pour une communication et/ou une alimentation d'énergie, en particulier pour une transmission filaire par un câble ou une transmission sans fil.

13. Procédé de fabrication d'un système de mesure médico-technique selon l'une des revendications précédentes 1 à 12, comprenant les étapes consistant à :
- fournir une conduite (20) qui est adaptée pour guider un fluide, en particulier du sang,
- disposer un dispositif de détection (30) et facultativement des composantes supplémentaires du système de mesure sur la conduite (20) notamment sur une surface périphérique extérieure (22.1) d'une paroi de la conduite (20),
- disposer le système de mesure avec la conduite (20) dans un outil, et
- surmouler au moins le dispositif de détection (30) et la conduite (20), respectivement au moins en partie, en particulier avec une matière à base de polyester et de résine hydrocarbonée.

14. Procédé selon la revendication 13, dans lequel le surmoulage est exécuté en deux étapes, en surmoulant d'abord dans une première étape au moins le dispositif de détection (30), en particulier de manière à ce que le dispositif de détection (30) soit disposé dans un agencement fonctionnel, et en surmoulant ensuite dans une seconde étape en outre au moins la conduite (20), dans lequel le surmoulage (9) est appliqué de préférence dans un moule final, et avant le surmoulage, de préférence un mandrin est introduit dans la conduite (20).

15. Procédé selon l'une des revendications précédentes, dans lequel, lors de la mise en place dans l'outil, un positionnement relatif du dispositif de détection (30) par rapport à l'outil est effectué, en particulier par rapport à un moule négatif pour former un passage dans le surmoulage (9), et dans lequel la conduite (20), lors de la mise en place dans l'outil, est fixée de préférence à au moins un réceptacle de l'outil, en particulier à un étrier de retenue ou à une bride de retenue d'un moule négatif de l'outil.
